(19) Europäisches Patentamt
European Patent Office
Office européen des brevets

(11) EP 0 501 322 B1

(12) EUROPEAN PATENT SPECIFICATION

(45) Date of publication and mention of the grant of the patent: **04.09.1996 Bulletin 1996/36**

(51) Int. Cl.$^6$: **C07D 401/12**, A61K 31/40

(21) Application number: **92102900.5**

(22) Date of filing: **21.02.1992**

# (54) 3-Piperidinylmethylcarboxylate substituted indoles

Substituierte Piperidimylmethyl-Ester von 3-Indolcarbonsäure

Esters pipéridimylmethyl substitués d'acide indole-3-carbonyligne

(84) Designated Contracting States:
**AT BE CH DE DK ES FR GB GR IT LI LU MC NL PT SE**

(30) Priority: **25.02.1991 GB 9103862**

(43) Date of publication of application:
**02.09.1992 Bulletin 1992/36**

(73) Proprietor: **GLAXO GROUP LIMITED
Greenford, Middlesex UB6 0NN (GB)**

(72) Inventors:
- **Oxford, Alexander William
Ware, Hertfordshire SG12 ODG (GB)**
- **Whitehead, John William Frank
Ware, Hertfordshire SG12 ODG (GB)**
- **Knight, John
Ware, Hertfordshire SG12 ODG (GB)**

(74) Representative: **Filler, Wendy Anne, Dr. et al
Glaxo Wellcome plc
Glaxo Wellcome House,
Berkeley Avenue
Greenford, Middlesex UB6 0NN (GB)**

(56) References cited:
**EP-A- 0 144 986**
**EP-A- 0 229 391**
**EP-A- 0 230 718**
**GB-A- 2 152 049**

Remarks:
The file contains technical information submitted after the application was filed and not included in this specification

EP 0 501 322 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.13.4/3.4

## Description

This invention relates to indole derivatives, to processes for their preparation, to pharmaceutical compositions containing them and to their medical use.

British Patent Specification No. 2152049 describes inter alia compounds of formula

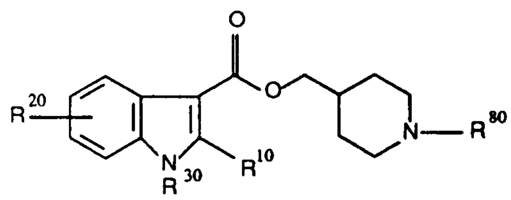

wherein
$R^{10}$ and $R^{20}$ independently represent hydrogen, halogen, $C_{1-4}$alkyl, $C_{1-4}$alkoxy, hydroxy, amino, $C_{1-4}$alkylamino, di$C_{1-4}$alkylamino, mercapto or $C_{1-4}$alkylthio;
$R^{30}$ represents hydrogen, $C_{1-4}$alkyl, $C_{3-5}$alkenyl, aryl or aralkyl; and $R^{80}$ represents hydrogen, $C_{1-7}$alkyl, $C_{3-5}$alkenyl or benzyl.

These compounds are stated to exhibit serotonin M receptor antagonist activity.

European Patent Specification No. 229391 describes inter alia compounds of formula

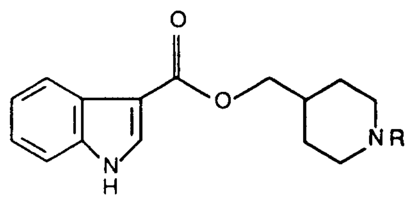

wherein
R represents hydrogen, lower alkyl, optionally substituted benzyl, optionally substituted benzoyl, pyridyl, 2-hydroxyethyl, pyridylmethyl or

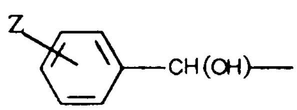

where Z represents halogen, which compounds are claimed to be of use in preventing dementias and sequelae of cerebrovascular diseases.

The present invention relates to novel compounds which are potent and specific antagonists of 5-hydroxytryptamine (5-HT;serotonin).

Thus, the present invention provides an indole derivative of formula (I):

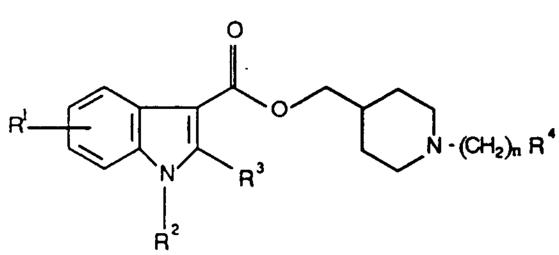

(I)

wherein

$R^1$ represents a hydrogen or a halogen atom, or a $C_{1-6}$alkyl, $C_{1-6}$alkoxy or hydroxy group;
$R^2$ represents a hydrogen atom or a $C_{1-6}$alkyl, $-CH_2C_{2-5}$alkenyl or $-CH_2C_{2-5}$alkynyl group;
$R^3$ represents a hydrogen atom or a $C_{1-6}$alkyl or $C_{1-6}$alkoxy group;
n represents 2 or 3;
$R^4$ represents a group selected from cyano, hydroxyl, $C_{1-6}$alkoxy, phenoxy, $C(0)C_{1-6}$alkyl, $C(0)C_6H_5$, $-CONR^5R^6$, $-NR^5COR^6$, $-SO_2NR^5R^6$ or $-NR^5SO_2R^6$ (wherein each of $R^5$ and $R^6$ independently represent a hydrogen atom, a $C_{1-6}$alkyl or phenyl group);

and quaternary ammonium derivatives, piperidine N-oxides and pharmaceutically acceptable salts and solvates thereof.

Suitable pharmaceutically acceptable salts of the compounds of general formula (I) include acid addition salts formed with pharmaceutically acceptable organic or inorganic acids for example, hydrochlorides, hydrobromides, sulphates, alkyl-or arylsulphonates (e.g. methanesulphonates or p-toluenesulphonates), phosphates, acetates, citrates, succinates, tartrates, fumarates and maleates.

Other acids, such as oxalic, while not in themselves pharmaceutically acceptable, may be useful in the preparation of salts useful as intermediates in obtaining the compounds of the invention and their pharmaceutically acceptable acid addition salts. The solvates may, for example, be hydrates.

References hereafter to a compound according to the invention includes both compounds of formula (I) and their quaternary ammonium derivatives, piperidine N-oxides and pharmaceutically acceptable acid addition salts together with pharmaceutically acceptable solvates.

Quaternary ammonium derivatives of compounds of formula (I) are compounds of formula

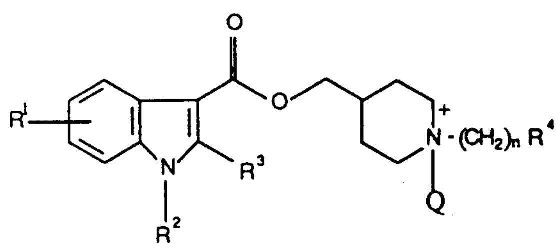

where Q represents $C_{1-6}$alkyl (e.g. methyl).

Piperidine N-oxides of compounds of formula (I) are compounds of formula

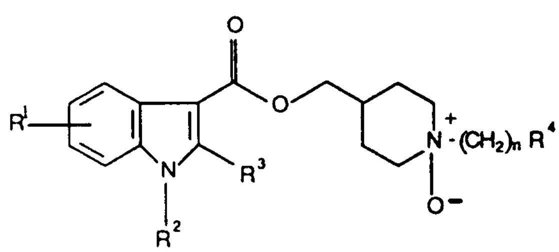

All optical isomers of compounds of general formula (I) and their mixtures including the racemic mixtures thereof, and all the geometric isomers of compounds of formula (I), are embraced by the invention.

Referring to the general formula (I), a $C_{1-6}$alkyl group may be a straight chain or branched chain alkyl group, for example, methyl, ethyl, propyl, prop-2-yl, butyl, but-2-yl, 2-methylprop-2-yl, pentyl, pent.3.yl or hexyl. A $-CH_2C_{2-5}$alkenyl group may be, for example, a propenyl or butenyl group. A $-CH_2C_{2-5}$alkynyl group may be, for example, a prop-2-ynyl group. When $R^1$ represents a halogen atom this may be, for example, a fluorine, chlorine, bromine or iodine atom. $R^1$ may be attached at either the 4-, 5-, 6- or 7- (e.g. the 4-, 5-, or 7- more preferably the 5- or 7-, most preferably the 5-) position of the indole ring.

A preferred class of compounds of formula (I) is that in which $R^4$ is a group selected from cyano, $C_{1-6}$alkoxy, phenoxy, $C(0)C_{1-6}$alkyl, $C(0)C_6H_5$, $-CONR^5R^6$, $-NR^5COR^6$, $-SO_2NR^5R^6$ or $-NR^5SO_2R^6$ (wherein each of $R^5$ and $R^6$ independently represent a hydrogen atom, a $C_{1-6}$alkyl or phenyl group);

Another preferred class of compounds formula (I) is that in which $R^4$ represents a group -CN, hydroxy, $-C_{1-6}$alkoxy (e.g. methoxy), $-CONR^5R^6$ (e.g. $CONH_2$), $-NR^5COR^6$ (e.g. NHCOMe), $-SO_2NR^5R^6$ (e.g. $SO_2NHMe$) or $-NR^5SO_2R^6$ (e.g. $NHSO_2Me$ or $NHSO_2Ph$).

Another preferred class of compounds of formula (I) is that in which $R^4$ represents a group -CN, $-C_{1-6}$alkoxy (e.g. methoxy), $-CONR^5R^6$ (e.g. $CONH_2$), $-NR^5COR^6$ (e.g. NHCOMe), $-SO_2NR^5R^6$ (e.g. $SO_2NHMe$) or $-NR^5SO_2R^6$ (e.g.$NHSO_2Me$ or $NHSO_2Ph$).

A further preferred class of compounds of formula (I) is that in which $R^1$ represents a hydrogen atom, a halogen (e.g. fluorine) atom or a $C_{1-6}$alkyl (e.g. methyl) group. Furthermore, when $R^1$ represents a halogen (e.g. fluorine) atom or a $C_{1-6}$alkyl (e.g. methyl) group this is preferably attached at the 5-, or 7- position (e.g. the 5-position) of the indole ring.

Another preferred class of compounds of formula (I) is that in which $R^2$ represents a hydrogen atom or a $C_{1-6}$alkyl (e.g. methyl) group.

Yet another preferred class of compounds of formula (I) is that in which $R^3$ represents a hydrogen atom or a $C_{1-6}$alkoxy (e.g. methoxy) group.

Another preferred class of compounds of formula (I) is that in which n represents 2.

In a preferred aspect, the present invention provides compounds of formula (Ia)

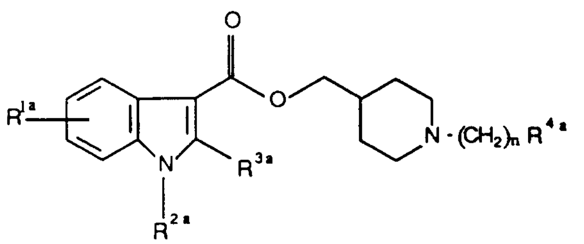

(Ia)

wherein

$R^{1a}$ represents a hydrogen or a halogen atom, or a $C_{1-6}$alkyl, or $C_{1-6}$alkoxy group;
$R^{2a}$ represents a hydrogen atom or a $C_{1-6}$alkyl group;
$R^{3a}$ represents a hydrogen atom or a $C_{1-6}$alkyl or $C_{1-6}$alkoxy group;
n represents 2 or 3;
$R^{4a}$ represents a group selected from CN, $-C_{1-6}$alkoxy, $-C(O)C_{1-6}$alkyl, $-CONR^{5a}R^{6a}$, $-NR^{5a}COR^{6a}$, $-SO_2NR^{5a}R^{6a}$ or $-NR^{5a}SO_2R^{6a}$ (wherein each of $R^{5a}$ and $R^{6a}$ independently represent a hydrogen atom or a $C_{1-6}$alkyl group); and quaternary ammonium derivatives, piperidine N-oxides and pharmaceutically acceptable salts and solvates thereof.

A preferred class of compound of formula (Ia) is that in which $R^{1a}$ is attached at the 4-, 5- or 7- (e.g. the 5- or 7-, most preferably the 5-) position of the indole ring.

A further preferred class of compounds of formula (Ia) is that in which $R^{1a}$ represents a hydrogen or a halogen (e.g. fluorine) atom or a $C_{1-6}$alkyl (e.g. methyl) group. Furthermore, when $R^{1a}$ represents a halogen (e.g. fluorine) atom or a $C_{1-6}$alkyl (e.g. methyl) group this is preferably attached at the 5-, or 7- position (e.g. the 5-position) of the indole ring.

Another preferred class of compounds of formula (Ia) is that in which $R^{2a}$ represents a hydrogen atom or a methyl group.

Yet another preferred class of compounds of formula (Ia) is that in which $R^{3a}$ represents a hydrogen atom or a $C_{1-6}$alkoxy (e.g. methoxy) group.

Another preferred class of compounds of formula (Ia) is that in which n represents 2.

Another preferred class of compounds of formula (Ia) is that in which $R^{4a}$ represents a group -CN, $-C_{1-6}$alkoxy (e.g. methoxy), $-CONR^{5a}R^{6a}$ (e.g. $CONH_2$), $-NR^{5a}COR^{6a}$ (e.g.NHCOMe), $-SO_2NR^{5a}R^{6a}$ (e.g.$SO_2NHMe$) or $-NR^{5a}SO_2R^{6a}$ (e.g.$NHSO_2Me$ or $NHSO_2Ph$).

A preferred group of compounds of formula (Ia) is that in which $R^{1a}$ represents a hydrogen or halogen (e.g. fluorine) atom or a $C_{1-6}$alkyl (e.g. methyl) group (e.g. a fluorine atom or a methyl group in the 5-position of the indole ring), $R^{2a}$ represents a hydrogen atom or a $C_{1-6}$alkyl (e.g. methyl) group, $R^{3a}$ represents a hydrogen atom or a $C_{1-6}$alkoxy (e.g. methoxy) group, n represents 2, and $R^{4a}$ represents a group $-S0_2NR^{5a}R^{6a}$(e.g.$SO_2NHMe$) or $NR^{5a}SO_2R^{6a}$ (e.g.$NHSO_2Me$ or $NHSO_2Ph$).

Specific compounds according to the invention are:-

[1-[2-[(Methylsulphonyl)amino]ethyl]-4-piperidinyl]methyl 1-methyl-1H- indole-3-carboxylate;

[1-(2-Hydroxyethyl)-4-piperidinyl]methyl 1-methyl-1H-indole-3- carboxylate;
[1-[2-[(Methylamino)sulphonyl]ethyl]-4-piperidinyl]methyl 1-methyl-1H-indole-3-carboxylate;
[1-(2-Methoxyethyl)-4-piperidinyl]methyl 1-methyl-1H-indole-3-carboxylate;
[1-(3-Amino-3-oxopropyl)-4-piperidinyl]methyl 1-methyl-1H-indole-3-carboxylate;
[1-(2-Cyanoethyl)-4-piperidinyl]methyl 1-methyl-1H-indole-3-carboxylate;
[1-[2-[(Methylsulphonyl)amino]ethyl]-4-piperidinyl]methyl 1H-indole-3-carboxylate;
[1-[2-[(Methylsulphonyl)amino]ethyl]-4-piperidinyl]methyl 2-methoxy-1H-indole-3-carboxylate;
[1-[2-(Acetylamino)ethyl]-4-piperidinyl]methyl 1-methyl-1H-indole-3-carboxylate;
[1-[3-[(Methylsulphonyl)amino]propyl]-4-piperidinyl]methyl 1-methyl-1H-indole-3-carboxylate;
[1-[2-[(Methylsulphonyl)amino]ethyl]-4-piperidinyl]methyl 5-fluoro-1H-indole-3-carboxylate;
[1-[2-[(Methylsulphonyl)amino]ethyl]-4-piperidinyl]methyl 5-fluoro-2-methoxy-1H-indole-3-carboxylate;
[1-[2-[(Methylsulphonyl)amino]ethyl]-4-piperidinyl]methyl 2-methoxy-1-methyl-1H-indole-3-carboxylate;
[1-[2-[(Phenylsulphonyl)amino]ethyl]-4-piperidinyl]methyl 1-methyl-1H-indole-3-carboxylate;
[1-[2-[(Methylsulphonyl)amino]ethyl]-4-piperidinyl]methyl 5-fluoro-1-methyl-1H-indole-3-carboxylate;
[1-[2-[(Methylsulphonyl)amino]ethyl]-4-piperidinyl]methyl 5-methyl-1H-indole-3-carboxylate;
[1-[2-[(Methylsulphonyl)amino]ethyl]-4-piperidinyl]methyl 2-methoxy-5-methyl-1H-indole-3-carboxylate;
[1-[2-[Methyl(methylsulphonyl)amino]ethyl]-4-piperidinyl]methyl 1-methyl-1H-indole-3-carboxylate;
[1-[2-[(Methylsulphonyl)amino]ethyl]-4-piperidinyl]methyl 5-fluoro-2-methoxy-1H-indole-3-carboxylate N-oxide;
1-Methyl-4-[[[(1-methyl-1H-indol-3-yl)carbonyl]oxy]methyl]-1-[2-[(methylsulphonyl)amino]ethyl]piperidinium iodide;
4-[[[(2-Methoxy-1H-indol-3-yl)carbonyl]oxy]methyl]-1-methyl-1-2-[2-[(methylsulphonyl)amino]ethyl]piperidinium iodide; and pharmaceutically acceptable salts, more particularly the hydrochloride, methanesulphonate and maleate salts, and solvates thereof.

The compounds of the invention are antagonists of 5-HT both in vitro and in vivo and are thus of use in the treatment of conditions mediated by 5-HT.

In particular the compounds of the invention inhibit the 5-HT induced contraction of guinea-pig colon (essentially following the general procedure described by C.J. Elswood et.al in Br.J. Pharmac., 1990, 100, (Proc.Suppl.) 485P and Eur. J. Pharmac., 1991, 196, 149-155 in the presence of ondansetron and methysergide) and the 5-HT-induced secretion in rat colon (as described by K T Bunce et.al in Br.J. Pharmac., 1991, 102, 811-816), and are thus useful in the treatment of 5-HT mediated disorders in which there is a disturbance of gastrointestinal function. Conditions involving disturbance of intestinal function include for example irritable bowel syndrome and its associated pain, excessive gastrointestinal secretion, and/or diarrhoea for example diarrhoea associated with excessive gastrointestinal secretion, cholera infection and carcinoid syndrome. The compounds of the invention may also be useful in the treatment of emesis.

The compounds of the invention have been shown to be $5\text{-}HT_4$ antagonists in vitro as demonstrated by their ability to inhibit the 5-HT-induced relaxation of the rat oesophagus preparation (essentially following the general procedure described by J J Reeves et al in Br. J Pharmac., 1989, 98, (Proc.Suppl.), 800P and 1991, 103, 1067-1072) and are thus of use in the treatment of conditions capable of amelioration by antagonism of such receptors. $5\text{-}HT_4$ receptors have been found in for example the digestive and urinary tracts, brain and cardiovascular system of mammals including man and are thus believed to be associated with conditions involving the digestive and urinary tracts (e.g. urinary incontinence), cardiovascular system and CNS disorders.

Thus the compounds of the invention may also be useful in the treatment of movement disorders (e.g. Parkinsonism), psychoses such as schizophrenia, mania, dementia or other cognitive disorders e.g. Alzheimer's disease; depression; and dependency on drugs or substances of abuse.

The compounds of the invention have been shown to be active in the rat social interaction test as described by B J Jones et al in Br J Pharmac., 1988, 93, 985-93 and are thus of use in the treatment of anxiety.

In a further aspect the invention therefore provides a compound of formula (I) or a quaternary ammonium derivative, piperidine N-oxide or a pharmaceutically acceptable salt or solvate thereof for use in therapy, in particular in human medicine. It will be appreciated that use in therapy embraces but is not necessarily limited to use of a compound of the invention as an active therapeutic substance.

There is also provided as a further aspect of the invention the use of a compound of formula (I) or a quatenary ammonium derivative, piperidine N-oxide or a pharmaceutically acceptable salt or solvate thereof in the preparation of a medicament for use in the treatment of conditions mediated by 5-hydroxytryptamine, in particular conditions capable of amelioration by antagonism of $5\text{-}HT_4$ receptors.

In an alternative or further aspect there is provided a method for the treatment of a mammal, including man, comprising administration of an effective amount of a compound of formula (I) or a quaternary ammonium derivative, piperidine N-oxide or a pharmaceutically acceptable salt or solvate thereof in particular in the treatment of conditions mediated by, 5-hydroxytryptamine, in particular conditions capable of amelioration by antagonism of $5\text{-}HT_4$ receptors.

It will be appreciated that reference to treatment is intended to include prophylaxis as well as the alleviation of established symptoms. Compounds according to the invention may be administered as the raw chemical but the active ingredient is preferably presented as a pharmaceutical formulation.

Accordingly, the invention also provides a pharmaceutical composition comprising a compound of formula (I) or a quaternary ammonium derivative, piperidine N-oxide or a pharmaceutically acceptable salt or solvate thereof and formulated for administration by any convenient route. Such compositions are preferably in a form adapted for use in medicine, in particular human medicine, and can conveniently be formulated in conventional manner using pharmaceutically acceptable carriers.

Thus the compounds according to the invention may be formulated for oral, buccal, parenteral, topical, implant or rectal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds of the invention may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal and pulmonary administration, the compounds according to the invention may be formulated as solutions or suspensions for administration via a suitable metered or unit dose device or alternatively as a powder mix with a suitable carrier for administration using a suitable delivery device.

For topical and percutaneous administration, the compounds according to the invention may be formulated as solutions, suspensions, creams or ointments and be included in systems that provide controlled release.

A proposed dose of the compounds of the invention for administration to man (of approximately 70kg body weight) is 1mg to 100mg, of the active ingredient per unit dose expressed as the weight of free base, which could be administered, for example, 1 to 4 times per day. It will be appreciated that it may be necessary to make routine variations to the dosage, depending on the age and condition of the patient and the precise dosage will be ultimately at the discretion of the attendant physician or veterinarian. The dosage will also depend on the route of administration.

The compounds of the invention may, if desired, be administered in combination with one or more other therapeutic agents and formulated for administration by any convenient route in a conventional manner. Appropriate doses will be readily appreciated by those skilled in the art.

Compounds of formula (I), and quaternary ammonium derivatives, piperidine N-oxides and pharmaceutically acceptable salts or solvates thereof, may be prepared by the general methods outlined hereinafter. In the following description, the groups $R^1$ to $R^4$ and n are as previously defined for compounds of formula (I) unless otherwise stated.

According to a first general process (A), a compound of formula (I) may be prepared by reacting a compound of formula (II):

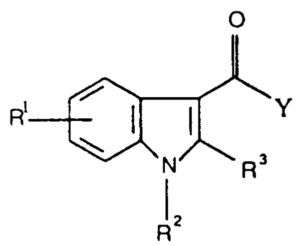

(II)

or a protected derivative thereof, wherein Y represents a leaving atom or group such as a halogen (e.g. chlorine) atom or an imidazolide group, or a group $R^7CO_2$-(wherein $R^7$ represents an alkyl or fluoroalkyl group such as trifluoromethyl or t-butyl), with a compound of formula (III):

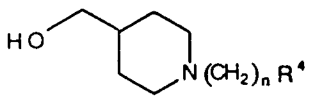

(III)

or an alkali metal (e.g. lithium) alkoxide thereof, optionally in the presence of a strong acid such as methanesulphonic acid.

The reaction is conveniently effected in an inert organic solvent such as an ether (e.g. tetrahydrofuran) or a halogenated hydrocarbon (e.g. dichloromethane) at a temperature between -80°C and the reflux temperature of the solvent.

According to another general process (B), a compound of formula (I) may be prepared by alkylating a compound of formula (IV):

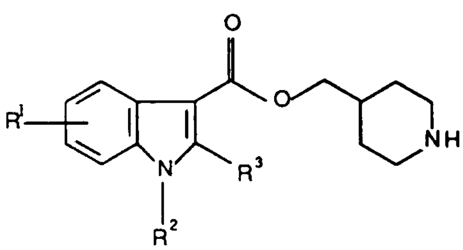

(IV)

or a protected derivative thereof, with a compound of formula (V):

$$L(CH_2)_nR^4 \qquad (V)$$

wherein L represents a leaving atom or group such as a halogen (e.g. bromine or iodine) atom, or an acyloxy (e.g. trifluoroacetyloxy) or a sulphonyloxy (e.g. p-toluenesulphonyloxy) group; or a protected derivative thereof, in the presence of a base such as a tertiary amine (e.g. diisopropylethylamine or triethylamine).

The reaction is conveniently effected in an inert organic solvent such as acetonitrile, a substituted amide (e.g. dimethylformamide) or an aromatic hydrocarbon (e.g. toluene), at an elevated temperature, for example at the reflux temperature of the solvent.

According to another general process (C), a compound of formula (I) may be converted into another compound of formula (I) using conventional techniques.

Thus, for example, a compound of formula (I) where $R^3$ represents a $C_{1-6}$alkoxy (e.g. methoxy) group may be prepared by reacting a compound of formula (I) wherein $R^3$ represents a hydrogen atom with N-chlorosuccinimide followed by an appropriate alcohol (e.g. methanol). The reaction conveniently takes place in a suitable solvent such as chlorinated hydrocarbon (e.g. chloroform) at ambient temperature.

In addition, compounds of formula (I) where $R^2$ is a hydrogen atom may be converted into another compound of formula (I) wherein $R^2$ represents a $C_{1-6}$alkyl (e.g. methyl) group with a suitable alkylating agent such as an alkyliodide (e.g. methyliodide).

Similarly, compounds of formula (I) where $R^4$ represents a group containing an -NH- moiety may be converted into another compound of formula (I) wherein $R^4$ contains a -$N(C_{1-6}$alkyl)- moiety (e.g. -$N(CH_3)$-) using a suitable alkylating

agent as described above. The reaction conveniently takes place in a suitable solvent such as an ether (e.g. tetrahydrofuran) at ambient temperature and in the presence of a strong base such as sodium hydride.

Also, quaternary ammonium, salts of compounds of formula (I) may be prepared by reacting non-quaternary compounds of formula (I) with a suitable quaternising agent such as Q-L (where L is a leaving group as defined above e.g. a halogen (e.g. iodine) atom and Q is as defined above). The reaction conveniently takes place in a suitable solvent such as a chlorinated hydrocarbon (e.g. chloroform) at ambient temperature.

Piperidine N-oxides of compounds of formula (I) may be prepared by reacting an appropriate piperidine compound of formula (I) with a suitable oxidising agent such as 3-chloro peroxybenzic acid. Oxidation conveniently takes place in a suitable solvent such as a halogenated hydrocarbon (e.g. chloroform) at ambient temperature.

It should be appreciated that in the above transformations it may be necessary or desirable to protect any sensitive groups in the molecule of the compound in question to avoid undesirable side reactions. For example, it may be necessary to protect the indole nitrogen atom, for example with an arylmethyl (e.g. trityl), alkyl (e.g. t-butyl), alkoxymethyl (e.g. methoxymethyl), acyl (e.g. benzyloxycarbonyl) or a sulphonyl (e.g. N,N-dimethylaminosulphonyl or p-toluenesulphonyl) group. When $R^4$ represents a hydroxyl group it may be necessary to protect the hydroxyl group, for example with an arylmethyl (e.g. benzyl or trityl) group.

It may be necessary to protect compounds where $R^4$ contains a sensitive group such as an amine group. Such groups may be protected for example using an acyl group (e.g. benzyloxycarbonyl) or a silyl group (e.g. trimethylsilyl).

Thus according to another general process (D), a compound of general formula (I) may be prepared by the removal of any protecting groups from a protected form of a compound of formula (I). Deprotection may be effected using conventional techniques such as those described in 'Protective Groups in Organic Synthesis' by T. W. Greene (John Wiley and Sons, 1981).

For example, considering the N-protecting groups first, a trityl group may be cleaved by acid hydrolysis (e.g. using dilute hydrochloric or acetic acid). An alkoxyalkyl group may be removed using a mineral acid (e.g. dilute hydrochloric acid). An acyl (e.g. benzyloxycarbonyl) group may be removed by hydrolysis under acidic or basic conditions (e.g. using hydrogen bromide or sodium hydroxide or, where milder conditions are required, sodium carbonate or cesium carbonate). A sulphonyl group may be removed by alkaline hydrolysis. An arylmethyl OH-protecting group may be cleaved under acidic or basic conditions (e.g. with dilute acetic acid, hydrobromic acid or boron tribromide) or by hydrogenolysis in the presence of a catalyst (e.g. palladium on charcoal).

Compounds of formula (II) may be prepared, for example, by reacting a compound of formula (VI):

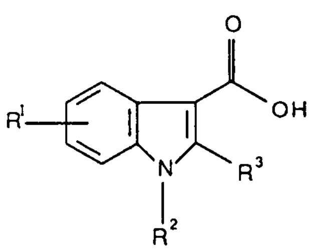

(VI)

with a suitable halogenating reagent (e.g. oxalyl chloride or thionyl chloride), an imidazole derivative (e.g. N,N-carbonyldiimidazole), or an anhydride (e.g. trifluoroacetic or t-butyl anhydride).

Compounds of formula (IV) may be prepared, for example, by reacting a compound of formula (VII)

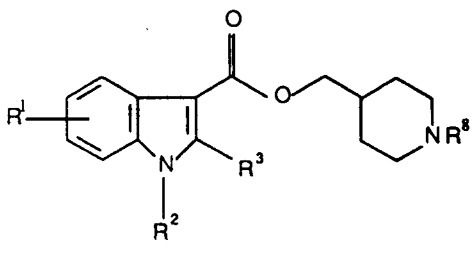

(VII)

wherein $R^8$ is a $C_{1-6}$alkyl (e.g. methyl) group with a chloroformate (e.g. a-chloroethylchloroformate, vinylchloroformate or ethylchloroformate) at an elevated temperature, with subsequent heating of the reaction mixture at reflux temperature with an alcohol (e.g. methanol).

Compounds of formula (VII) may be prepared according to the method of general process (A), by reacting a compound of formula (II) with a compound of formula (VIII)

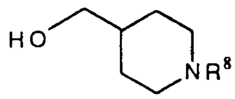

(VIII)

in which $R^8$ is a $C_{1-6}$alkyl (e.g. methyl) group.

Compounds of formulae (III), (V), (VI) and (VIII) are either known, or may be prepared from known compounds by conventional procedures.

In addition, compounds of formula (III) may be prepared by reduction of the corresponding compounds of formula (IX)

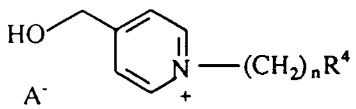

(IX)

wherein $A^-$ represents an associated anion such as a halide (e.g. bromide) anion. Reduction may be conveniently effected by hydrogenation in the presence of a suitable catalyst, such as rhodium on alumina, in the presence of a suitable solvent, for example under aqueous conditions.

Compounds of formula (IX) may be prepared by alkylation of 4-pyridine methanol using a suitable alkylating agent of formula (V) as defined hereinbefore. The reaction conveniently takes place in the presence of sodium iodide in a suitable solvent such as an alcohol (e.g. isopropanol) at the reflux temperature of the solvent.

Where it is desired to isolate a compound of the invention as a salt, for example a physiologically acceptable salt, this may be achieved by reacting the compound of formula (I) in the form of the free base with an appropriate acid, preferably with an equivalent amount, in a suitable solvent such as an alcohol (e.g. ethanol or methanol), an ester (e.g. ethyl acetate) or an ether (e.g. tetrahydrofuran).

Physiologically acceptable salts may also be prepared from other salts, including other physiologically acceptable salts, of the compound of formula (I) using conventional methods.

The compounds of the invention may readily be isolated in association with solvent molecules by crystallisation from or evaporation of an appropriate solvent to give the corresponding solvates.

Individual enantiomers of the compounds of the invention may be obtained by resolution of a mixture of enantiomers (e.g a racemic mixture) using conventional means, such as an optically active resolving acid; see for example 'Stereochemistry of Carbon Compounds' by E.L.Eliel (McGraw Hill, 1962) and 'Tables of Resolving Agents' by S. H. Wilen.

The various general methods described above may be used for the introduction of the desired groups at any stage in the stepwise formation of the required compound, and it will be appreciated that these general methods can be combined in different ways in such multi-stage processes. The sequence of the reactions in multi-stage processes should of course be chosen so that the reaction conditions used do not affect groups in the molecule which are desired in the final product.

The invention is further illustrated by the following Intermediates and Examples. All temperatures are in °C. Thin layer chromatography (t.l.c.) was carried out on silica, and flash column chromatography (FCC) on silica (Merck 9385). Solvent System A as used for chromatography denotes dichloromethane:ethanol:0.88 ammonia solution. Organic extracts were dried, where indicated, over magnesium sulphate.

Intermediate 1

(1-Methyl-4-piperidinyl)methyl 1-methyl-1H-indole-3-carboxylate

1-Methylindole-3-carboxylic acid (1.74g) was suspended in dry dichloromethane (50ml). Oxalyl chloride (0.9ml) was added and the mixture was stirred for 1h. The solvent was removed in vacuo to leave the acid chloride as a solid. 4-Hydroxymethyl-1-methylpiperidine (1.40g) in dry THF (20ml) was cooled to -78° under nitrogen. n-Butyllithium (1.64M; 6.0ml) was added, and the solution was stirred for 1h. The acid chloride in dry THF (20ml) was added, and the mixture was allowed. to warm to room temperature over 2h. The mixture was then diluted with ether (200ml), washed with 8% aqueous sodium bicarbonate solution (200ml), dried, and the solvent was removed in vacuo to leave a solid which was triturated with ether to give the title compound (1.25g), m.p. 102-104°.

Intermediate 2

4-Piperidinylmethyl 1-methyl-1H-indole-3-carboxylate

(1-Methyl-4-piperidinyl)methyl 1-methyl-1H-indole-3-carboxylate (2.40g) and α-chloroethylchloroformate (15ml) were heated together at 50° for 2h. Methanol (20ml) was then added, and the mixture was heated at reflux for 0.5h. The solvent was removed in vacuo to leave a solid which was purified by FCC eluting with System A (100:8:1) to give the title compound (1.43g), m.p. 126-130°.

Intermediate 3

N-[2-[4-(Hydroxymethyl)-1-piperidinyl]ethyl]methanesulphonamide

4-Piperidine methanol (1.60g) was dissolved in dry acetonitrile (40ml), N,N-diisopropylethylamine (5ml) was added, followed by N-(2-bromoethyl)methanesulphonamide (2.95g) in acetonitrile (10ml), and the resulting mixture heated at reflux for 2h. The solvent was removed in vacuo to leave a gum. This was purified by FCC eluting with System A (75:8:1) to give the title compound (1.80g) as a solid, m.p. 81-82°.

Intermediate 4

[1-[2-[(Methylsulphonyl)[(phenylmethoxy)carbonyl]amino]ethyl]-4-piperidinyl]methyl 2-methoxy-1H-indole-3-carboxylate

[1-[2-[(Methylsulphonyl)amino]ethyl]-4- piperidinyl]methyl 2-methoxy-1H-indole-3-carboxylate (440mg) was dissolved in acetonitrile (10ml) and 4-N,N-dimethylaminopyridine (654mg) was added, followed by benzylchloroformate (0.61ml) and the mixture stirred at room temperature for 18h. 6.5% Methanolic ammonia (10ml) was added, and the mixture stirred for 1h. The mixture was poured into 2M sodium carbonate solution (100ml), extracted with ethyl acetate (2x100ml), dried and the solvent evaporated in vacuo to leave a pale yellow gum. FCC eluting with System A (300:10:1) gave the title compound (359mg) a white foam.
T.l.c. (System A, 300:10:1) Rf 0.32

Intermediate 5

[1-[2-[(Methylsulphonyl)[(phenylmethoxy)carbonyl]amino]ethyl]-4-piperidinyl]methyl 2-methoxy-1-methyl-1H-indole-3-carboxylate

[1-[2-[(Methylsulphonyl)[(phenylmethoxy)carbonyl]amino]ethyl]-4-piperidinyl]methyl 2-methoxy-1H-indole-3-carboxylate (327mg) was dissolved in dry THF (6ml). Tetra n-butylammonium fluoride 1M solution in THF (2.4ml) was added, followed by methyliodide (0.15ml) and the resulting solution stirred for 30min. The mixture was poured into 2M sodium carbonate solution (100ml), extracted with ethyl acetate (2x100ml), dried and the solvent removed in vacuo to leave a brown foam. FCC eluting with ether:cyclohexane:triethylamine (30:10:4) gave the title compound (130mg) as a white foam.
T.l.c. (ether:cyclohexane:triethylamine, 30:10:4) Rf 0.19

Intermediate 6

[1-(Phenylmethyl)-4-piperidinyl]methyl 1H-indole-3-carboxylate

1H-Indole-3-carboxylic acid (3.0g) was suspended in dry dichloromethane (60ml) under nitrogen. Oxalyl chloride (2ml) was added, and the mixture stirred for 2h. The solvent was removed in vacuo to leave the acid chloride as an orange solid 1-Benzyl-4-hydroxymethylpiperidine (4.11g) in dry tetrahydrofuran (60ml) was cooled to -78° under nitrogen, n-butyl lithium 1.64M (11.3ml) was added, and the mixture stirred for 30min. The acid chloride in dry tetrahydrofuran (60ml) was added dropwise, and the resulting solution stirred for 1h at -78° then allowed to warm to room temperature over 2h. The mixture was diluted with dichloromethane (250ml), washed with 2M potassium carbonate solution (2x250ml), dried, and the solvent removed in vacuo to leave a brown gum. FCC using System A (300:8:1) as eluant gave a pale yellow solid which was triturated with cyclohexane:ether (2:1) to give the title compound (4.5g) as a white solid, m.p. 106°-107.5°.

Intermediate 7

(4-Piperidinyl)methyl 1H-indole-3-carboxylate

[1-(Phenylmethyl)-4-piperidinyl]methyl 1H-indole-3-carboxylate (2.0g) in ethanol (60ml) was added to a pre-hydrogenated suspension of palladium on charcoal 10% (100mg) in ethanol (50ml) and the mixture stirred under an atmosphere of hydrogen for 24h. The mixture was filtered, and the filtrate evaporated in vacuo to leave the title compound as a white solid, (1.45g) m.p. 185°-187°.

Intermediate 8

4-(Hydroxymethyl) - 1 - [2-[(methylsulphonyl)amino]ethyl]pyridinium bromide

A stirred solution of 4-pyridine-methanol (90g) and N-2-bromoethylmethane sulphonamide (207g) with sodium iodide (12.6g) in isopropanol (2000ml) was heated under reflux, under nitrogen for 5 days. The mixture was cooled to room temperature. The solid material was then collected, washed with isopropanol (2 x 100 ml) and dried in vacuo at 50-55° for 10h. The title compound (231.2g) was obtained as a white crystalline solid. The material was then recrystallised from methanol (1200ml) to give the title compound (219g) m.p. 176-17°.

Intermediate 9

N-[2-[4-(Hydroxymethyl)-1-piperidinyl]ethyl]methanesulphonamide

A solution of 4-(Hydroxymethyl) - 1 - [2-[(methylsulphonyl) amino]ethyl]pyridinium bromide (120g) in water (1700ml) was hydrogenated in the presence of 5% rhodium on alumina (12g) for (5x8h). The catalyst was filtered off and washed with water (ca. 200ml). The solvent was then removed in vacuo. The residual oil (140g) was then purified by FCC using System A (50:8:1) as the eluent. After a small amount of the fast running material was collected the solvent ratio was increased to a 25:8:1 mixture. The title compound (62g) was isolated as a near colourless oil which crystallised under vacuo to give a colourless crystalline solid, m.p. 86-88°.

Example 1

[1-[2-[(Methylsulphonyl)amino]ethyl]-4-piperidinyl]methyl 1-methyl-1H- indole-3-carboxylate

To a solution of 4-piperidinylmethyl 1-methyl-1H-indole-3- carboxylate (200mg) in dry acetonitrile (6ml) was added diisopropylethylamine (0.26ml) followed by N-(2-bromoethyl)-methanesulphonamide (178mg), and the mixture was heated at reflux for 2h. The cooled mixture was poured into 2M sodium hydroxide solution (150ml), extracted with ether (2x150ml), dried, and the solvent was removed in vacuo to leave a gum which was purified by FCC eluting with System A (200:8:1) to give the title compound (86mg), m.p. 95-97°.

| | | | | |
|---|---|---|---|---|
| Analysis $C_{19}H_{27}N_3O_4S$ | Found: | C,57.8; | H,7.0; | N,10.4; |
| | requires | C,58.0; | H,6.9; | N,10.7%. |

Example 2

[1-(2-Hydroxyethyl)-4-piperidinyl]methyl 1-methyl-1H-indole-3- carboxylate

To a solution of 4-piperidinylmethyl 1-methyl-1H-indole-3- carboxylate (200mg) in acetonitrile (6ml) was added diisopropylethylamine (0.19ml), followed by 2-iodoethanol (0.07ml), and the mixture was heated at reflux overnight. The solvent was then removed in vacuo, and the residue was partitioned between ether (80ml) and 2M sodium hydroxide (50ml). The organic phase was dried and the solvent was removed in vacuo to leave a solid which was crystallised from ether to give the title compound (51mg), m.p. 127-128°, t.l.c. (System A, 200:8:1) Rf 0.10.

Example 3

[1-[2-(Methylamino)sulphonyl]ethyl]-4-piperidinyl]methyl 1-methyl-1H-indole-3-carboxylate maleate

A mixture of 4-piperidinylmethyl 1-methyl-1H-indole-3-carboxylate (200mg), diisopropylethylamine (0.25ml), and N-methylethene sulphonamide (107mg), in dry acetonitrile (6ml) was heated at reflux under nitrogen for 2h. The solvent was then removed in vacuo to give a solid (310mg) which was purified by FCC eluting with System A (400:10:1) to give the free base of the title compound (250mg) as a solid, m.p. 128-129°, t.l.c. (System A, 400:10:1) Rf 0.33.
A sample of the free base (220mg) was dissolved in dichloromethane (2ml) and treated with a solution of maleic acid (68mg) in absolute ethanol (1ml). The resultant solid was filtered off and washed with dry ether (3x20ml) to give the title compound (280mg), m.p. 170-171°.

| | | | | |
|---|---|---|---|---|
| Analysis $C_{19}H_{27}N_3O_4S.C_4H_4O_4$ | Found: | C,54.3; | H,6.2; | N,8.2; |
| | requires | C,54.2; | H,6.1; | N,8.3% |

Example 4

[1-(2-Methoxyethyl)-4-piperidinyl]methyl 1-methyl-1H-indole-3-carboxylate

A mixture of 4-piperidinylmethyl 1-methyl-1H-indole-3-carboxylate (200mg), diisopropylethylamine (0.25ml), and bromoethyl methylether (0.08m) in dry acetonitrile (6ml) was heated at reflux under nitrogen for 2h. The solvent was then removed in vacuo to give an oil (340mg), which was purified by FCC eluting with System A (400:10:1) to give the title compound (210mg) as a solid, m.p. 151-153°, t.l.c. (System A, 400:10:1) Rf 0.35.

Example 5

[1-(3-Amino-3-oxopropyl)-4-piperidinyl]methyl 1-methyl-1H-indole-3-carboxylate

A mixture of 4-piperidinylmethyl 1-methyl-1H-indole-3-carboxylate (250mg), diisopropylethylamine (0.32ml), and acrylamide (78mg), in dry acetonitrile (7.5ml) was heated at reflux under nitrogen for 24h. The solvent was then removed in vacuo to give an oil (355mg) which was purified by FCC eluting with System A (200:10:1) to give the title compound (295mg) as a solid, m.p. 126-127°.

| | | | | |
|---|---|---|---|---|
| Analysis $C_{19}H_{25}N_3O_3$ | Found | C,66.1; | H,7.6; | N,11.8; |
| | requires | C,66.5; | H,7.3; | N,12.2%. |

Example 6

[1-(2-Cyanoethyl)-4-piperidinyl]methyl 1-methyl-1H-indole-3-carboxylate maleate

A mixture of 4-piperidinylmethyl 1-methyl-1H-indole-3-carboxylate (750mg), diisopropylethylamine (0.96ml) and acrylonitrile (0.22ml) in dry acetonitrile (20ml) was heated at reflux under nitrogen for 24h. The solvent was then removed in vacuo to give an oil (880mg) which was purified by FCC eluting with System A (500:10:1) to give the free base of the title compound (825mg) as a solid, m.p. 94-95°.
A sample of the free base (175mg) was dissolved in dichloromethane (2ml) and treated with a solution of maleic acid (68mg), in absolute ethanol (1ml). The solvent was removed in vacuo and the residue was triturated with dry ether (3x5ml) to give the title compound (235mg) as a solid, m.p. 174-175°.

| | | | | |
|---|---|---|---|---|
| Analysis $C_{19}H_{23}N_3O_2.C_4H_4O_4$ | Found: | C,62.7; | H,6.2; | N,9.4; |
| | requires | C,62.6; | H,6.2; | N,9.5%. |

Example 7

[1-[2-(Methylsulphonyl)amino]ethyl]-4-piperidinyl]methyl 1H-indole-3-carboxylate

4-Piperidinylmethyl 1H-indole-3-carboxylate (1.31g) was dissolved in hot acetonitrile (50ml). Diisopropylethylamine (1.76ml) was added followed by N-(2-bromoethylmethanesulphonamide) (1.0g) and the resulting mixture was stirred at room temperature for ca. 72h. The solvent was removed in vacuo and the residue was dissolved in dichloromethane (250ml). The solution was then washed with 2M potassium carbonate solution (2x250ml), dried, and solvent was removed in vacuo to leave a gum, which was purified by FCC eluting with System A (150:8:1) to give the title compound as a solid, m.p. 114-116°.

| | | | | |
|---|---|---|---|---|
| $C_{18}H_{25}N_3O_4S$ | Assayfound: | C,57.2; | N,6.7; | N,10.9; |
| | requires | C,57.0; | H,6.6; | N,11.1%. |

Example 8

[1-[2-(Methylsulphonyl)amino]ethyl]-4-piperidinyl]methyl 2-methoxy-1H-indole-3-carboxylate

To a solution of [1-[2-(methylsulphonyl)amino]ethyl]-4-piperidinyl]methyl 1H-indole-3-carboxylate (1.0g) in chloroform (30ml) was added N chlorosuccinimide (525mg), and the resulting solution was stirred for 3h. Methanol (5ml) was added and the solution was stirred overnight. The mixture was then diluted with dichloromethane (200ml), washed with 2M sodium carbonate solution (200ml), dried and the solvent was removed in vacuo to leave a gum which was purified by FCC (200:8:1) to give a solid (410mg). Crystallisation of this solid twice from methanol:ether (1:10) gave the title compound (230mg), m.p. 131.5-134°, t.l.c. (System A, 200:8:1) Rf O.38.

Example 9

[1-[2-(Acetylamino)ethyl]-4-piperidinyl]methyl 1-methyl-1H-indole-3-carboxylate maleate

A mixture of 4-piperidinylmethyl 1-methyl-1H-indole-3-carboxylate (250mg), diisopropylethylamine (0.32ml), and N-(2-chloroethyl) acetamide (0.11ml) in dry acetonitrile (7.5ml) was heated at reflux under nitrogen for 48h. The solvent was then removed in vacuo to give an oil (ca. 550mg) which was purified by FCC eluting with System A (200:10:1) to give an oil (127mg). This material was dissolved in dichloromethane (2ml) and treated with a solution of maleic acid (43mg), in absolute ethanol (1ml). The solvent was removed in vacuo and the residue was triturated with dry ether (5x5ml) to give the title compound (150mg) as a solid, m.p. 151-152°.

| | | | | |
|---|---|---|---|---|
| Analysis $C_{20}H_{27}N_3O_3.C_4H_4O_4$ | Found: | C,60.8; | N,6.6; | N,8.6; |
| | requires | C,60.9; | H,6.6; | N,8.9%. |

Example 10

[1-[3-[(Methylsulphonyl)amino]propyl]-4-piperidinyl]methyl 1-methyl-1H-indole-3-carboxylate maleate

A mixture of 4-piperidinylmethyl 1-methyl-1H-indole-3-carboxylate (250mg), diisopropylethylamine (0.32ml) and N-(3-bromopropyl)methanesulphonamide (238mg) in dry acetronitrile (7.5ml) was heated at reflux under nitrogen for 2.5h. The solvent was removed in vacuo to give an oil (ca.625mg), which was purified by FCC eluting with System A (200:10:1) to give an oil (200mg). This material was dissolved in dichloromethane (3ml) and treated with a solution of maleic acid (60mg) in absolute ethanol (1ml). The solvent was removed in vacuo and the residue was triturated with dry ether (3 x 10ml) to give the title compound (250mg) as a solid, m.p. 159-160°.

| | | | | |
|---|---|---|---|---|
| Analysis $C_{20}H_{29}N_3O_4S.C_4H_4O_4$ | Found: | C,55.1; | H,6.4; | N,7.9% |
| | requires | C,55.1; | H,6.4; | N,8.0% |

Example 11

[1-[2-[(Methylsulphonyl)amino]ethyl]-4-piperidinyl]methyl 5-fluoro-1H-indole-3-carboxylate

Trifluoroacetic anhydride (0.47ml) was added dropwise to a stirred suspension of 5-fluoroindole-3-carboxylic acid (500mg) in dry dichloromethane (25ml) under nitrogen. After 1h the suspension was treated with methane sulphonic acid (0.22ml) followed immediately with a suspension of N-[2-[4-(hydroxymethyl)-1-piperidinyl]ethyl]methanesulphonamide (792mg) in dry dichloromethane (20ml). The solution was stirred for 2.5h then poured into 8% sodium bicarbonate solution (100ml). The organic layer was separated and the aqueous phase extracted with dichloromethane (2 x 50ml). The combined organic extracts were dried, filtered and evaporated to give an oil (21.1g). This was purified by FCC eluting with System A (250:1O:1) to give the title compound (635mg) as a foam, t.l.c. (System A, 250:10:1) Rf 0.17.

| | | | | |
|---|---|---|---|---|
| Analysis $C_{18}H_{24}FN_3O_4S$ | Found: | C,53.9; | H,6.2; | N,10.1% |
| | requires | C,54.4; | H,6.1; | N,10.6%. |

Example 12

[1-[2-[(Methylsulphonyl)amino]ethyl]-4-piperidinyl]methyl 5-fluoro-2-methoxy-1H-indole-3-carboxylate hydrochloride

N-chlorosuccinimide (302mg) was added to a stirred solution of [1-[2-[(methylsulphonyl)amino]ethyl]-4-piperidinyl methyl 5-fluoro-1H-indole-3-carboxylate (600mg) in chloroform (20ml) under nitrogen. After 3h the solvent was removed in vacuo and the residue treated with methanol (10ml). The solution was stirred under nitrogen for 1h and the solvent removed in vacuo to give an oil (950mg). This was purified by FCC eluting with System A (200:10:1) to give a foam (395mg). This material was dissolved in dichloromethane/ethanol (5ml) and treated with an excess of ethereal hydrogen chloride solution. The solvent was removed in vacuo and the residue triturated with dry ether (5 x 10ml) to give the title compound (400mg) as a solid, m.p. 207-209°.

| | | |
|---|---|---|
| Analysis | Found: | C,49.1; H,6.0; N,8.8% |
| $C_{19}H_{26}FN_3O_5S.HCl$ | requires | C,49.2; H,5.9; N,9.1%. |

Example 13

[1-[2-[(Methylsulphonyl)amino]ethyl]-4-piperidinyl]methyl 2-methoxy-1-methyl-1H-indole-3-carboxylate

[1-[2-[(Methylsulphonyl)[(phenylmethoxy)carbonyl]amino]ethyl]-4-piperidinyl]methyl 2-methoxy-1-methyl-1H-indole-3-carboxylate (130mg) was dissolved in dry THF (1ml) and methanol (2ml). Cesium carbonate (75mg) was added and the mixture stirred for 1h. The mixture was poured into 2M sodium carbonate solution (100ml), extracted with ethyl acetate (2x100ml), dried and the solvent removed in vacuo to leave a yellow gum. FCC eluting with System A (300:8:1) gave the title compound (65mg) as a white crystalline solid, m.p.103-104°.
T.l.c. (System A, 300:8:1) Rf 0.26.

Example 14

[1-[2-[(Phenylsulphonyl)amino]ethyl]-4-piperidinyl]methyl 1-methyl-1H-indole-3-carboxylate

To 4-piperidinylmethyl 1-methyl-1H-indole-3-carboxylate (0.5g) in acetonitrile (30ml) was added diisopropylethylamine (0.48ml) followed by N-(2-bromoethyl)benzenesulphonamide (0.48g) and the reaction mixture heated at reflux for 3h. The cooled reaction mixture was diluted with dichloromethane (200ml) and washed with 2M sodium carbonate (200ml), dried and the solvent removed in vacuo to give a yellow oil. This was purified by FCC eluting with System A (200:8:1). The solvent was removed in vacuo to give the free base as an amorphous white foam (0.64g). Ethyl acetate (20ml) was added whereupon crystallisation occurred, the mixture was diluted with ether, filtered and dried in vacuo to give the title compound (0.508g) as an off-white crystalline solid m.p. >240°.
γmax (Nujol) 3125, 1691, 1536, 953, 745, 694 $cm^{-1}$

| | | | | |
|---|---|---|---|---|
| $C_{24}H_{29}N_3O_4S$ | Assay Found: | C,63.6; | H,6.4; | N,9.2; |
| | requires: | C,63.3; | H,6.4; | N,9.2% |

Example 15

[1-[2-[(Methylsulphonyl)amino]ethyl]-4-piperidinyl]methyl 5-fluoro-1-methyl-1H-indole-3-carboxylate hydrochloride

Trifluoroacetic anhydride (0.32ml) was added dropwise to a stirred suspension of 5-fluoro-1-methylindole-3-carboxylic acid (220mg) in dry dichloromethane (10ml) under nitrogen. After 10min the brown solution was treated with methane sulphonic acid (0.15ml) followed immediately with a suspension of N-[2-[-4-(hydroxymethyl)-1-piperidinyl]ethyl]methanesulphonamide (539mg) in dry dichloromethane (15ml). The solution was stirred for 1h, poured into 8% sodium bicarbonate solution (50ml) and extracted with dichloromethane (3x25ml). The combined extracts were dried, filtered and evaporated to give a brown oil (550mg). FCC with System A (250:10:1) as the eluent gave a pink foam (300mg). This material was dissolved in dichloromethane (5ml) and treated with an excess of ethereal hydrogen chloride solution. The solvent was removed in vacuo and the residue triturated with dry ether (5x10ml) to give the title compound as a pink solid (320mg) m.p. 223-4°
T.l.c. (System A 250:10:1), Rf 0.22
Water assay Found 0.44%w/w=0.11mol.$H_2O$

Similarly prepared:-

Example 16

[1-[2-[(Methylsulphonyl)amino]ethyl]-4-piperidinyl]methyl 5-methyl-1H-indole-3-carboxylate (1.28g), T.l.c. System A (75:8:1), Rf 0.53

| | | | | |
|---|---|---|---|---|
| $C_{19}H_{27}N_3O_4S.0.4H_2O$ | Assay Found: | C,57.0; | H,7.2; | N,10.3; |
| | requires: | C,57.0; | H,7.0; | N,10.5% |

Water assay indicates presence of 0.8mol equiv. of water.
From 5-methyl-1H-indole-3-carboxylic acid (1.00g).

Example 17

[1-[2-[(Methylsulphonyl)amino]ethyl]-4-piperidinyl]methyl 2-methoxy-5-methyl-1H-indole-3-carboxylate

N-Chlorosuccinimide (0.18g) was added to a stirred solution of [1-[2-[(methylsulphonyl)amino]ethyl]-4-piperidinyl]methyl 5-methyl-1H-indole-3-carboxylate (0.35g) in chloroform (10ml) under nitrogen. After stirring at room temperature for 4.75h, the solvent was removed in vacuo and the residue was treated with methanol (5ml). The solution was stirred under nitrogen at room temperature for 1.5h, and the solvent was then removed in vacuo to give a yellow residue. After storing overnight in the refrigerator, the residue was purified by FCC eluting with System A (100:8:1). The appropriate fractions were combined and concentrated in vacuo to give the title compound (0.16g) as an off-white crystalline solid (0.16g) m.p.167-169°.

T.l.c. (System A 100:8:1), Rf 0.20

| | | | | |
|---|---|---|---|---|
| $C_{20}H_{29}N_3O_4S$ | Assay Found: | C,56.5; | H,7.1; | N,9.6; |
| | requires: | C,56.7; | H,6.9; | N,9.9% |

Example 18

1-Methyl-4-[[[(1-methyl-1H-indol-3-yl)carbonyl]oxy]methyl]-1-[2-[(methylsulphonyl)amino]ethyl]piperidinium iodide

A sample of [1-[2-[(methylsulphonyl)amino]ethyl]-4-piperidinyl] methyl 1-methyl-1H-indole-3-carboxylate maleate (0.5g) was partitioned between 2N sodium hydroxide (30ml) and chloroform (15ml). The organic layer was separated, the aqueous extracted twice with chloroform and the combined organic solution dried. After filtration the chloroform solution was treated with methyl iodide (1ml) and stirred at room temperature for 2 days. By t.l.c. (System A 75:8:1) there was still starting material present. The heterogeneous mixture was stirred under gentle reflux for 5h. The reaction mixture was filtered whilst still warm, the solid was washed with chloroform and dried in vacuo to give the title compound as a white solid (0.31g) with m.p. 186-189°.

| | | | | |
|---|---|---|---|---|
| Analysis $C_{20}H_{30}IN_3O_4S$ | Found: | C,44.8; | H,5.65; | N,7.7; |
| | requires | C,44.8; | H,5.65; | N,7.85% |

Example 19

4-[[[(2-Methoxy-1H-indol-3-yl)carbonyl]oxy]methyl]-1-methyl-1-2-[2-[(methylsulphonyl)amino]ethyl]piperidinium iodide

[1-[2-(Methylsulphonyl)amino]ethyl]-4-piperidinyl]methyl 2-methoxy-1H-indole-3-carboxylate hydrochloride (250mg) was treated with 8% sodium bicarbonate (10ml) and the aqueous extracted with chloroform (3x10ml). The combined organic extracts were dried and filtered. The solution was treated with methyl iodide (1ml) and stirred under nitrogen at 50° to 55° for 18h. Additional methyl iodide (1ml) was added and the solution stirred at reflux for 8h. The mixture was left to stand for 16h then concentrated in vacuo. The resultant cream foam was dissolved in hot isopropanol (ca. 60ml) and left to stand overnight. The solution was concentrated in vacuo (to ca. 10ml) and the residue collected by filtration. After drying in vacuo the title compound was obtained a cream foam (0.232g).γmax (Nujol) 3415, 3154, 1675, 1557 $cm^{-1}$

| | | | | |
|---|---|---|---|---|
| $C_{20}H_{13}IN_3O_5S.0.35C_3H_8O.0.7H_2O$ | Assay Found: | C,42.9; | H,5.7; | N,7.0; |
| | requires | C,43.2; | H,5.9; | N,7.2% |

Water assay Found 2.24%w/w water (0.7 mol equiv.)

Example 20

[1-[2-[Methyl(methylsulphonyl)amino]ethyl]-4-piperidinyl]methyl 1-methyl-1H-indole-3-carboxylate

[1-[2-(Methylsulphonyl)amino]ethyl-4-piperidinyl]methyl 1-methyl-1H-indole-3-carboxylate (700mg) was dissolved in dry tetrahydrofuran (50ml). Sodium hydride 70% dispersion in mineral oil (73mg) was added and the mixture stirred for 1h. Methyl iodide (0.11ml) was added and the resulting mixture stirred under nitrogen for 5h. The mixture was poured into 2M sodium carbonate solution (200ml), extracted with ether (2x200ml), dried, and the solvent removed in vacuo to leave the title compound (420mg) as a white solid, m.p.=144°-145.5°.
T.l.c. System A (200:8:1), Rf 0.30

Example 21

[1-[2-[(Methylsulphonyl)amino]ethyl]-4-piperidinyl]methyl 5-fluoro-2-methoxy-1H-indole-3-carboxylate methanesulphonate

A solution of [1-[2-[(methylsulphonyl)amino]ethyl]-4-piperidinyl] methyl 5-fluoro-2-methoxy-1H-indole-3-carboxylate (500mg) in absolute ethanol (15ml) was treated with a solution of methanesulphonic acid (113mg) in absolute ethanol (2ml). The solvent was removed in vacuo and the oily residue triturated with dry ether (3x50ml) to give the title compound as a white solid (610mg) m.p. 184-185°.
T.l.c. System A (150:10:1), Rf 0.24

Example 22

[1-[2-[(Methylsulphonyl)amino]ethyl]-4-piperidinyl]methyl 5-fluoro-2-methoxy-1H-indole-3-carboxylate maleate

A solution of [1-[2-[(methylsulphonyl)amino]ethyl]-4-piperidinyl]methyl 5-fluoro-2-methoxy-1H-indole-3-carboxylate (500mg) in absolute ethanol (15ml) was treated with asolution of maleic acid (137mg) in absolute ethanol (2ml). The solvent was removed in vacuo and the oily residue triturated with dry ether (3x50ml) to give the title compound as a white solid (635mg), m.p. 96-7°.
T.l.c. System A (150:10:1), Rf 0.24.

Example 23

[1-[2-[(Methylsulphonyl)amino]ethyl]-4-piperidinyl]methyl 5-fluoro-2-methoxy-1H-indole-3-carboxylate N-oxide

3-Chloroperoxybenzoic acid (505mg) was added to a stirred solution of 1-[(2-sulphonamidomethyl)ethyl]-4-piperidinylmethyl 5-fluoro-1H-indole-3-carboxylate (500mg) in dry chloroform (25ml) under nitrogen. After 16h 0.1N hydrochloric acid (25ml) was added and the organic layer separated. The aqueous was washed with chloroform (25ml) then evaporated in vacuo to give a yellow oil (~600mg). FCC with System A (25:10:1) as the eluent gave a yellow oil which was triturated with dry ether (3x25ml) to give impure title compound (150mg) as a pale yellow solid, m.p. 113-6° (foamed). The above material was crystallised from isopropanol to give the title compound as a pale brown solid (48mg).

| | | | | |
|---|---|---|---|---|
| Analysis $C_{19}H_{26}FN_3O_6S.0.75^iPrOH$ | found: | C,52.3; | H,6.8; | N,8.3; |
| | requires: | C,52.2; | H,6.6; | N,8.6% |

Pharmacy Examples

Example 1 - Tablets

| | |
|---|---|
| a) Compound of the invention | 5.0mg |
| Lactose | 95.0mg |
| Microcrystlline Cellulose | 90.0mg |
| Cross-linked polyvinylpyrrolidone | 8.0mg |
| Magnesium Stearate | 2.0mg |
| Compression weight | 200.0mg |

The compound of the invention. microcrystalline cellulose, lactose and cross linked polyvinylpyrrolidone are sieved through a 500 micron sieve and blended in a suitable mixer. The magnesium stearate is sieved through a 250 micron sieve and blended with the active blend. The blend is compressed into tablets using suitable punches.

| | |
|---|---|
| b) Compound of the invention | 5.0mg |
| Lactose | 165.0mg |
| Pregelatinised Starch | 20.0mg |
| Cross-linked polyvinylpyrrolidone | 8.0mg |
| Magnesium Stearate | 2.0mg |
| Compression weight | 200.0mg |

The compound of the invention, lactose and pregelatinised starch are blended together and granulated with water. The wet mass is dried and milled. The magnesium stearate and cross-linked polyvinylpyrrolidone are screened through a 250 micron sieve and blended with the granule. The resultant blend is compressed using suitable tablet punches.

Example 2 - Capsules

| | |
|---|---|
| a) Compound of the invention | 5.0mg |
| Pregelatinised Starch | 193.0mg |
| Magnesium Stearate | 2.0mg |
| Fill weight | 200.0mg |

The compound of the invention and pregelatinised starch are screened through a 500 micron mesh sieve, blended together and lubricated with magnesium stearate, (meshed through a 250 micron sieve). The blend is filled into hard gelatine capsules of a suitable size.

| | |
|---|---|
| b) Compound of the invention | 5.0mg |
| Lactose | 177.0mg |
| Polyvinylpyrrolidone | 8.0mg |
| Cross-linked polyvinylpyrrolidone | 8.0mg |
| Magnesium Stearate | 2.0mg |
| Fill weight | 200.0mg |

The compound of the invention and lactose are blended together and granulated with a solution of polyvinylpyrrolidone. The wet mass is dried and milled. The magnesium stearate and cross-linked polyvinylpyrrolidone are screened through a 250 micron sieve and blended with the granules. The resultant blend is filled into hard gelatine capsules of a suitable size.

Example 3 - Syrup

| | |
|---|---|
| a) Compound of the invention | 5.0mg |
| Hydroxypropyl Methylcellulose | 45.0mg |
| Propyl Hydroxybenzoate | 1.5mg |
| Butyl Hydroxybenzoate | 0.75mg |
| Saccharin Sodium | 5.0mg |
| Sorbitol Solution | 1.0ml |
| Suitable Buffers | qs |
| Suitable flavours | qs |
| Purified Water to | 10.ml |

The hydroxypropyl methylcellulose is dispersed in a portion of hot purified water together with the hydroxybenzoates and the solution is allowed to cool to room temperature. The saccharin sodium flavours and sorbitol solution are added to the bulk solution. The compound of the invention is dissolved in a portion of the remaining water and added to the bulk solution. Suitable buffers may be added to control the pH in the region of maximum stability. The solution is made up to volume, filtered and filled into suitable containers.

Example 4 - Injection Formulation

| | % w/v |
|---|---|
| Compound of the invention | 1.00 |
| Water for injections B.P. to | 100.00 |

Sodium chloride may be added to adjust the tonicity of the solution and the pH may be adjusted to that of maximum stability and/or to facilitate solution of the compound of the invention using dilute acid or alkali or by the addition of suitable buffer salts. Antioxidants and metal chelating salts may also be included.

The solution is prepared, clarified and filled into appropriate sized ampoules sealed by fusion of the glass. The injection is sterilised by heating in an autoclave using one of the acceptable cycles. Alternatively the solution may be

sterilised by filtration and filled into sterile ampoules under aseptic conditions. The solution may be packed under an inert atmosphere of nitrogen.

## Claims

**Claims for the following Contracting States : AT, BE, CH, DE, DK, FR, GB, GR, IT, LI, LU, MC, NL, PT, SE**

**1.** Compounds of formula (I)

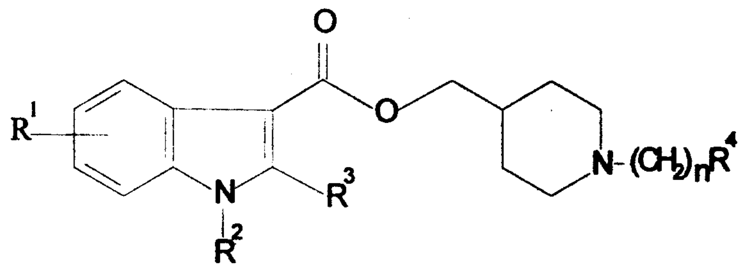

(I)

wherein

$R^1$ represents a hydrogen or a halogen atom, or a $C_{1-6}$alkyl, $C_{1-6}$alkoxy or hydroxy group;
$R^2$ represents a hydrogen atom or a $C_{1-6}$alkyl, $CH_2C_{2-5}$alkenyl or $CH_2C_{2-5}$alkynyl group;
$R^3$ represents a hydrogen atom or a $C_{1-6}$alkyl or $C_{1-6}$alkoxy group;
n represents 2 or 3;
$R^4$ represents a group selected from cyano, hydroxyl, $C_{1-6}$alkoxy phenoxy, $C(0)C_{1-6}$alkyl, $C(0)C_6H_5$, $CONR^5R^6$, $NR^5COR^6$, $SO_2NR^5R^6$ or $NR^5SO_2R^6$ (wherein each of $R^5$ and $R^6$ independently represent a hydrogen atom, a $C_{1-6}$alkyl or phenyl group);

and quaternary ammonium derivatives, piperidine N-oxides and pharmaceutically acceptable salts and solvates thereof.

**2.** A compound as claimed in claim 1 wherein the compound of formula (I) is a compound of formula (Ia)

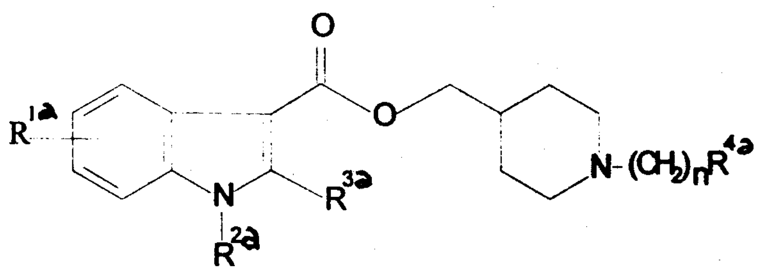

(Ia)

wherein

$R^{1a}$ represents a hydrogen or a halogen atom, or a $C_{1-6}$alkyl, or $C_{1-6}$alkoxy group;
$R^{2a}$ represents a hydrogen atom or a $C_{1-6}$alkyl group;
$R^{3a}$ represents a hydrogen atom or a $C_{1-6}$alkyl or $C_{1-6}$alkoxy group;
n represents 2 or 3;
$R^{4a}$ represents a group selected from CN, $C_{1-6}$alkoxy, $C(O)C_{1-6}$alkyl, $CONR^{5a}R^{6a}$, $NR^{5a}COR^{6a}$, $SO_2NR^{5a}R^{6a}$ or $NR^{5a}SO_2R^{6a}$ (wherein each of $R^{5a}$ and $R^{6a}$ independently represent a hydrogen atom or a $C_{1-6}$alkyl group);

and quaternary ammonium derivatives, piperidine N-oxides and pharmaceutically acceptable salts and solvates thereof.

3. A compound as claimed in claim 1 or claim 2 in which $R^1$ or $R^{1a}$ is in the 5-position of the indole ring.

4. A compound as claimed in any of claims 1 to 3 in which $R^1$ or $R^{1a}$ is a hydrogen or fluorine atom or a methyl group.

5. A compound as claimed in any of claims 1 to 4 in which $R^2$ or $R^{2a}$ is a hydrogen atom or a methyl group.

6. A compound as claimed in any of claims 1 to 5 in which $R^3$ or $R^{3a}$ is a hydrogen atom or a methoxy group.

7. A compound as claimed in any of claims 1 to 6 in which n is 2.

8. A compound as claimed in any of claims 1 to 7 in which $R^4$ or $R^{4a}$ is a group $NR^5SO_2R^6$ or $NR^{5a}SO_2R^{6a}$.

9. A compound as claimed in Claim 1 selected from

[1-[2-[(Methylsulphonyl)amino]ethyl]-4-piperidinyl]methyl 1-methyl-1H- indole-3-carboxylate;
(1-(2-Hydroxyethyl)-4-piperidinyl]methyl 1-methyl-1H-indole-3-carboxylate;
[1-[2-[(Methylamino)sulphonyl]ethyl]-4-piperidinyl]methyl 1-methyl-1H-indole-3-carboxylate;
[1-(2-Methoxyethyl)-4-piperidinyl]methyl 1-methyl-1H-indole-3-carboxylate;
[1-(3-Amino-3-oxopropyl)-4-piperidinyl]methyl 1-methyl-1H-indole-3-carboxylate;
[1-(2-Cyanoethyl)-4-piperidinyl]methyl 1-methyl-1H-indole-3-carboxylate;
[1-[2-[(Methylsulphonyl)amino]ethyl]-4-piperidinyl]methyl1H-indole-3-carboxylate;
[1-[2-[(Methylsulphonyl)amino]ethyl]-4-piperidinyl]methyl 2-methoxy-1H-indole-3-carboxylate;
[1-[2-(Acetylamino)ethyl]-4-piperidinyl]methyl 1-methyl-1H-indole-3-carboxylate;
[1-[3-[(Methylsulphonyl)amino]propyl]-4-piperidinyl]methyl 1-methyl-1H-indole-3-carboxylate;
[1-[2-[(Methylsulphonyl)amino]ethyl]-4-piperidinyl]methyl 5-fluoro-2-methoxy-1H-indole-3-carboxylate;
[1-[2-[(Methylsulphonyl)amino]ethyl]-4-piperidinyl]methyl-methoxy-1-methyl-1H-indole-3-carboxylate;
[1-[2-[(Phenylsulphonyl)amino]ethyl]-4-piperidinyl]methyl1-methyl-1H-indole-3-carboxylate;
[1-[2-[(Methylsulphonyl)amino]ethyl]-4-piperidinyl]methyl5-fluoro-1-methyl-1H-indole-3-carboxylate;
[1-[2-[(Methylsulphonyl)amino]ethyl]-4-piperidinyl]methyl5-methyl-1H-indole-3-carboxylate;
[1-[2-[(Methylsulphonyl)amino]ethyl]-4-piperidinyl]methyl2-methoxy-5-methyl-1H-indole-3-carboxylate;
[1-[2-[Methyl(methylsulphonyl)amino]ethyl]-4-piperidinyl]methyl1-methyl-1H-indole-3-carboxylate;
[1-[2-[(Methylsulphonyl)amino]ethyl]-4-piperidinyl]methyl 5-fluoro-2-methoxy-1H-indole-3-carboxylate N-oxide;
1-Methyl-4-[[[(1-methyl-1H-indol-3-yl)carbonyl]oxy]methyl]-1-[2-[(methylsulphonyl)amino]ethyl]piperidinium iodide;
4-[[[(2-Methoxy-1H-indol-3-yl)carbonyl]oxy]methyl]-1-methyl-1-2-[2-[(methylsulphonyl)amino]ethyl]piperidinium iodide; and pharmaceutically acceptable salts and solvates thereof.

10. [1-[2-[(Methylsulphonyl)amino]ethyl]-4-piperidinyl]methyl 5-fluoro-2-methoxy-1H-indole-3-carboxylate and pharmaceutically acceptable salts and solvates thereof.

11. The compound of claim 10 in the form of its hydrochloride salt.

12. The compound of claim 10 in the form of its methanesulphonate salt.

13. The compound of claim 10 in the form of its maleate salt.

14. A compound as claimed in any of claims 1 to 13 for use in therapy.

15. The use of a compound as claimed in any of claims 1 to 13 in the preparation of a medicament for use in the treatment of conditions mediated by 5-hydroxytryptamine.

16. A pharmaceutical composition comprising a compound as claimed in any of claims 1 to 13 together with a pharmaceutically acceptable carrier.

17. A process for the preparation of a compound of formula (I) as claimed in claim 1, which process comprises :-

(A) reacting a compound of formula (II):

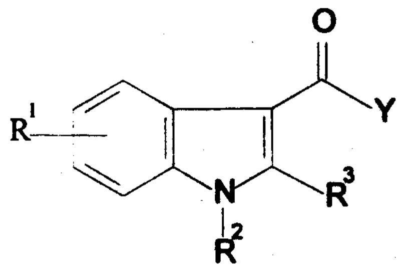

(II)

or a protected derivative thereof, wherein Y represents a leaving atom or group, with a compound of formula (III):

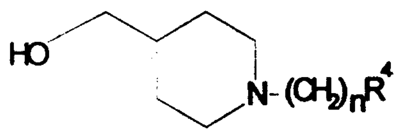

(III)

or an alkali metal alkoxide thereof, wherein $R^1$, $R^2$, $R^3$, $R^4$ and n are as defined in claim 1;

(B) alkylating a compound of formula (IV):

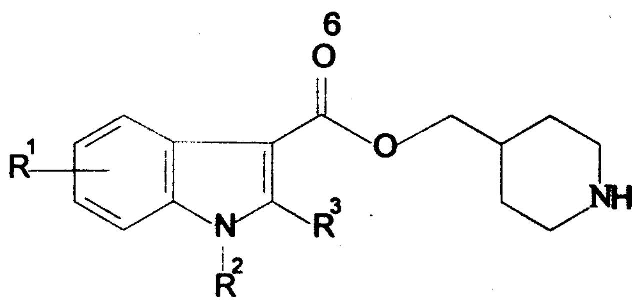

(IV)

or a protected derivative thereof, with a compound of formula (V)

$$L(CH_2)_nR^4 \qquad (V)$$

wherein L represents a leaving atom or group and $R^1$, $R^2$, $R^3$, $R^4$ and n are as defined in claim 1, or a protected derivative thereof, in the presence of a base;

(C) converting a compound of formula (I) into another compound of formula (I); or

(D) removing any protecting groups from a protected form of a compound of formula (I).

**Claims for the following Contracting State : ES**

1. A process for the preparation of compounds of formula (I)

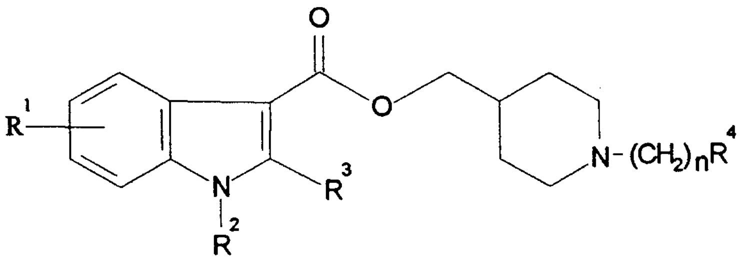

(I)

wherein

$R^1$ represents a hydrogen or a halogen atom, or a $C_{1-6}$alkyl, $C_{1-6}$alkoxy or hydroxy group;
$R^2$ represents a hydrogen atom or a $C_{1-6}$alkyl, $CH_2C_{2-5}$alkenyl or $CH_2C_{2-5}$alkynyl group;
$R^3$ represents a hydrogen atom or a $C_{1-6}$alkyl or $C_{1-6}$alkoxy group;
n represents 2 or 3;
$R^4$ represents a group selected from cyano, hydroxyl, $C_{1-6}$alkoxy, phenoxy, $C(0)C_{1-6}$alkyl, $C(0)C_6H_5$, $CONR^5R^6$, $NR^5COR^6$, $SO_2NR^5R^6$ or $NR^5SO_2R^6$ (wherein each of $R^5$ and $R^6$ independently represent a hydrogen atom, a $C_{1-6}$alkyl or phenyl group);

and quaternary ammonium derivatives, piperidine N-oxides and pharmaceutically acceptable salts and solvates thereof, which process comprises:-

(A) reacting a compound of formula (II):

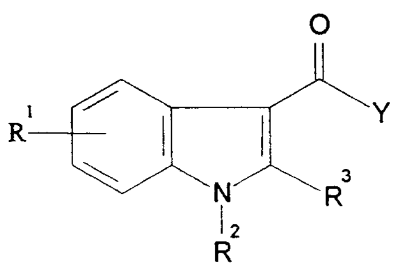

(II)

or a protected derivative thereof, wherein Y represents a leaving atom or group, with a compound of formula (III):

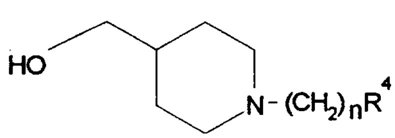

(III)

or an alkali metal alkoxide thereof, wherein $R^1$, $R^2$, $R^3$, $R^4$ and n are as defined above;

(B) alkylating a compound of formula (IV):

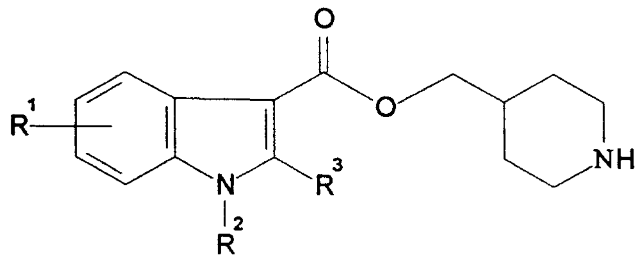

(IV)

or a protected derivative thereof, with a compound of formula (V)

$$L(CH_2)_nR^4 \quad (V)$$

wherein L represents a leaving atom or group and $R^1$, $R^2$, $R^3$, $R^4$ and n are as defined above, or a protected derivative thereof, in the presence of a base;

(C) converting a compound of formula (I) into another compound of formula (I); or

(D) removing any protecting groups from a protected form of a compound of formula (I).

2. A process according to claim 1 wherein the compound of formula (I) is a compound of formula (Ia)

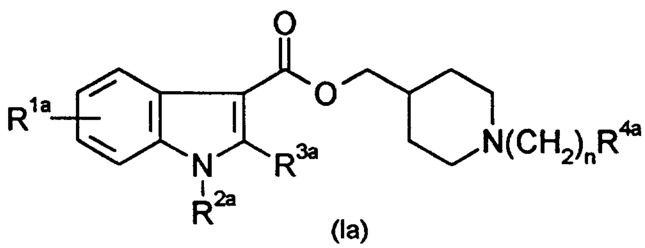

(Ia)

wherein

$R^{1a}$ represents a hydrogen or a halogen atom, or a $C_{1-6}$alkyl, or $C_{1-6}$alkoxy group;
$R^{2a}$ represents a hydrogen atom or a $C_{1-6}$alkyl group;
$R^{3a}$ represents a hydrogen atom or a $C_{1-6}$alkyl or $C_{1-6}$alkoxy group;
n represents 2 or 3;
$R^{4a}$ represents a group selected from CN, $C_{1-6}$alkoxy, $C(O)C_{1-6}$alkyl, $CONR^{5a}R^{6a}$, $NR^{5a}COR^{6a}$, $SO_2NR^{5a}R^{6a}$ or $NR^{5a}SO_2R^{6a}$ (wherein each of $R^{5a}$ and $R^{6a}$ independently represent a hydrogen atom or a $C_{1-6}$alkyl group);

and quaternary ammonium derivatives, piperidine N-oxides and pharmaceutically acceptable salts and solvates thereof.

3. A process as claimed in claim 1 or claim 2 in which $R^1$ or $R^{1a}$ is in the 5-position of the indole ring.

4. A process as claimed in any of claims 1 to 3 in which $R^1$ or $R^{1a}$ is a hydrogen or fluorine atom or a methyl group.

5. A process as claimed in any of claims 1 to 4 in which $R^2$ or $R^{2a}$ is a hydrogen atom or a methyl group.

6. A process as claimed in any of claims 1 to 5 in which $R^3$ or $R^{3a}$ is a hydrogen atom or a methoxy group.

7. A process as claimed in any of claims 1 to 6 in which n is 2.

8. A process as claimed in any of claims 1 to 7 in which $R^4$ or $R^{4a}$ is a group $NR^5SO_2R^6$ or $NR^{5a}SO_2R^{6a}$.

9. A process as claimed in Claim 1 for the preparation of a compound selected from

[1-[2-[(Methylsulphonyl)amino]ethyl]-4-piperidinyl]methyl 1-methyl-1H- indole-3-carboxylate;
(1-(2-Hydroxyethyl)-4-piperidinyl]methyl 1-methyl-1H-indole-3-carboxylate;
[1-[2-[(Methylamino)sulphonyl]ethyl]-4-piperidinyl]methyl 1-methyl-1H-indole-3-carboxylate;
[1-(2-Methoxyethyl)-4-piperidinyl]methyl 1-methyl-1H-indole-3-carboxylate;
[1-(3-Amino-3-oxopropyl)-4-piperidinyl]methyl 1-methyl-1H-indole-3-carboxylate;
[1-(2-Cyanoethyl)-4-piperidinyl]methyl 1-methyl-1H-indole-3-carboxylate;
[1-[2-[(Methylsulphonyl)amino]ethyl]-4-piperidinyl]methyl1H-indole-3-carboxylate;
[1-[2-[(Methylsulphonyl)amino]ethyl]-4-piperidinyl]methyl 2-methoxy-1H-indole-3-carboxylate;
[1-[2-(Acetylamino)ethyl]-4-piperidinyl]methyl 1-methyl-1H-indole-3-carboxylate;
[1-[3-[(Methylsulphonyl)amino]propyl]-4-piperidinyl]methyl 1-methyl-1H-indole-3-carboxylate;
[1-[2-[(Methylsulphonyl)amino]ethyl]-4-piperidinyl]methyl 5-fluoro-2-methoxy-1H-indole-3-carboxylate;
[1-[2-[(Methylsulphonyl)amino]ethyl]-4-piperidinyl]methyl-2-methoxy-1-methyl-1H-indole-3-carboxylate;
[1-[2-[(Phenylsulphonyl)amino]ethyl]-4-piperidinyl]methyl1-methyl-1H-indole-3-carboxylate;
[1-[2-[(Methylsulphonyl)amino]ethyl]-4-piperidinyl]methyl5-fluoro-1-methyl-1H-indole-3-carboxylate;
[1-[2-[(Methylsulphonyl)amino]ethyl]-4-piperidinyl]methyl5-methyl-1H-indole-3-carboxylate;
[1-[2-[(Methylsulphonyl)amino]ethyl]-4-piperidinyl]methyl2-methoxy-5-methyl-1H-indole-3-carboxylate;
[1-[2-[Methyl(methylsulphonyl)amino]ethyl]-4-piperidinyl]methyl1-methyl-1H-indole-3-carboxylate;
[1-[2-[(Methylsulphonyl)amino]ethyl]-4-piperidinyl]methyl 5-fluoro-2-methoxy-1H-indole-3-carboxylate N-oxide;
1-Methyl-4-[[[(1-methyl-1H-indol-3-yl)carbonyl]oxy]methyl]-1-[2-[(methylsulphonyl)amino]ethyl]piperidinium iodide;
4-[[[(2-Methoxy-1H-indol-3-yl)carbonyl]oxy]methyl]-1-methyl-1-2-[2-[(methylsulphonyl)amino]ethyl]piperidinium iodide; and pharmaceutically acceptable salts and solvates thereof.

10. A process according to claim 1 for the preparation of [1-[2-[(Methylsulphonyl)amino]ethyl]-4-piperidinyl]methyl 5-fluoro-2-methoxy-1H-indole-3-carboxylate and pharmaceutically acceptable salts and solvates thereof.

11. A process according to claim 1 for the preparation of the compound of claim 10 in the form of its hydrochloride salt.

12. A process according to claim 1 for the preparation of the compound of claim 10 in the form of its methanesulphonate salt.

13. A process according to claim 1 for the preparation of the compound of claim 10 in the form of its maleate salt.

14. The use of a compound of formula (I) in the preparation of a medicament for use in the treatment of conditions mediated by 5-hydroxytryptamine.

15. A process for the preparation of a pharmaceutical composition which comprises admixing a compound prepared according to claims 1 to 13 together with a pharmaceutically acceptable carrier.

**Patentansprüche**

**Patentansprüche für folgende Vertragsstaaten : AT, BE, CH, DE, DK, FR, GB, GR, IT, LI, LU, MC, NL, PT, SE**

1. Verbindungen der Formel (I):

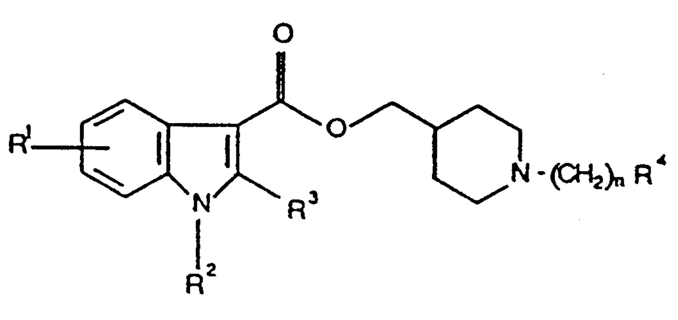

(I)

worin

$R^1$ ein Wasserstoff- oder ein Halogenatom oder eine $C_{1-6}$-Alkyl-, $C_{1-6}$-Alkoxy- oder Hydroxygruppe bedeutet;
$R^2$ ein Wasserstoffatom oder eine $C_{1-6}$-Alkyl-, $CH_2C_{2-5}$-Alkenyl- oder $CH_2C_{2-5}$-Alkinylgruppe bedeutet;
$R^3$ ein Wasserstoffatom oder eine $C_{1-6}$-Alkyl- oder $C_{1-6}$-Alkoxygruppe bedeutet;
n 2 oder 3 bedeutet;
$R^4$ eine Gruppe, ausgewählt aus Cyano, Hydroxyl, $C_{1-6}$-Alkoxy, Phenoxy, $C(O)C_{1-6}$-Alkyl, $C(O)C_6H_5$, $CONR^5R^6$, $NR^5COR^6$, $SO_2NR^5R^6$ oder $NR^5SO_2R^6$ bedeutet (wobei jeder der Substituenten $R^5$ und $R^6$ unabhängig ein Wasserstoffatom, eine $C_{1-6}$-Alkyl- oder Phenylgruppe bedeutet),

und die quaternären Ammoniumderivate, Piperidin-N-oxide und pharmazeutisch annehmbaren Salze und Solvate davon.

2. Verbindung nach Anspruch 1, wobei die Verbindung der Formel (I) eine Verbindung der Formel (Ia):

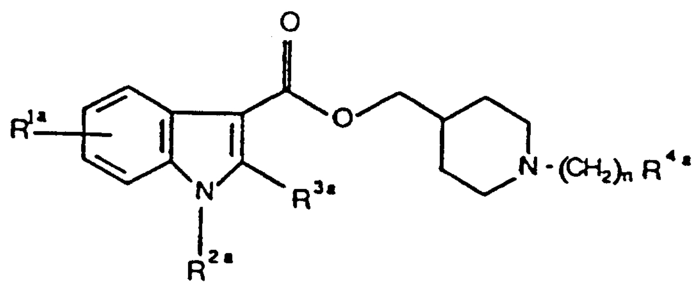

(Ia)

ist, worin

$R^{1a}$ ein Wasserstoff- oder ein Halogenatom oder eine $C_{1-6}$-Alkyl- oder $C_{1-6}$-Alkoxygruppe bedeutet;
$R^{2a}$ ein Wasserstoffatom oder eine $C_{1-6}$-Alkylgruppe bedeutet;
$R^{3a}$ ein Wasserstoffatom oder eine $C_{1-6}$-Alkyl- oder $C_{1-6}$-Alkoxygruppe bedeutet;
n 2 oder 3 bedeutet;
$R^{4a}$ eine Gruppe, ausgewählt aus CN, $C_{1-6}$-Alkoxy, , $C(O)C_{1-6}$-Alkyl, $CONR^{5a}R^{6a}$, $NR^{5a}COR^{6a}$, $SO_2NR^{5a}R^{6a}$ oder $NR^{5a}SO_2R^{6a}$ bedeutet (wobei jeder der Substituenten $R^{5a}$ und $R^{6a}$ unabhängig ein Wasserstoffatom oder eine $C_{1-6}$-Alkylgruppe bedeutet),

und die quaternären Ammoniumderivate, Piperidin-N-oxide und pharmazeutisch annehmbaren Salze und Solvate davon.

3. Verbindung nach Anspruch 1 oder 2, worin $R^1$ oder $R^{1a}$ in 5-Stellung des Indolrings steht.

4. Verbindung nach einem der Ansprüche 1 bis 3, worin $R^1$ oder $R^{1a}$ ein Wasserstoff- oder Fluoratom oder eine Methylgruppe bedeutet.

5. Verbindung nach einem der Ansprüche 1 bis 4, worin $R^2$ oder $R^{2a}$ ein Wasserstoffatom oder eine Methylgruppe bedeutet.

6. Verbindung nach einem der Ansprüche 1 bis 5, worin $R^3$ oder $R^{3a}$ ein Wasserstoffatom oder eine Methoxygruppe bedeutet.

7. Verbindung nach einem der Ansprüche 1 bis 6, worin n 2 bedeutet.

8. Verbindung nach einem der Ansprüche 1 bis 7, worin $R^4$ oder $R^{4a}$ eine Gruppe $NR^5SO_2R^6$ oder $NR^{5a}SO_2R^{6a}$ bedeutet.

9. Verbindung nach Anspruch 1, ausgewählt aus:

[1-[2-[(Methylsulfonyl)amino]ethyl]-4-piperidinyl]methyl-1-methyl-1H-indol-3-carboxylat;
[1-(2-Hydroxyethyl)-4-piperidinyl]methyl-1-methyl-1H-indol-3-carboxylat;
[1-[2-[(Methylamino)sulfonyl]ethyl]-4-piperidinyl]methyl-1-methyl-1H-indol-3-carboxylat;
[1-(2-Methoxyethyl)-4-piperidinyl]methyl-1-methyl-1H-indol-3-carboxylat;

[1-(3-Amino-3-oxopropyl)-4-piperidinyl]methyl-1-methyl-1H-indol-3-carboxylat;
[1-(2-Cyanoethyl)-4-piperidinyl]methyl-1-methyl-1H-indol-3-carboxylat;
[1-[2-[(Methylsulfonyl)amino]ethyl]-4-piperidinyl]methyl-1H-indol-3-carboxylat;
[1-[2-[(Methylsu1fonyl)amino]ethyl]-4-piperidinyl]methyl-2-methoxy-1H-indol-3-carboxylat;
[1-[2-(Acetylamino)ethyl]-4-piperidinyl]methyl-1-methyl-1H-indol-3-carboxylat;
[1-[3-[(Methylsulfonyl)amino]propyl]-4-piperidinyl]methyl-1-methyl-1H-indol-3-carboxylat;
[1-[2-[(Methylsulfonyl)amino]ethyl]-4-piperidinyl]methyl-5-fluor-2-methoxy-1H-indol-3-carboxylat;
[1-[2-[(Methylsulfonyl)amino]ethyl]-4-piperidinyl]methyl-2-methoxy-1-methyl-1H-indol-3-carboxylat;
[1-[2-[(Phenylsulfonyl)amino]ethyl]-4-piperidinyl]methyl-1-methyl-1H-indol-3-carboxylat;
[1-[2-[(Methylsulfonyl)amino]ethyl]-4-piperidinyl]methyl-5-fluor-1-methyl-1H-indol-3-carboxylat;
[1-[2-[(Methylsulfonyl)amino]ethyl]-4-piperidinyl]methyl-5-methyl-1H-indol-3-carboxylat;
[1-[2-[(Methylsulfonyl)amino]ethyl]-4-piperidinyl]methyl-2-methoxy-5-methyl-1H-indol-3-carboxylat;
[1-[2-[Methyl(methylsulfonyl)amino]ethyl]-4-piperidinyl]methyl-1-methyl-1H-indol-3-carboxylat;
[1-[2-[(Methylsulfonyl)amino]ethyl]-4-piperidinyl]methyl-5-fluor-2-methoxy-1H-indol-3-carboxylat-N-oxid;
1-Methyl-4-[[[(1-methyl-1H-indol-3-yl)carbonyl]oxy]methyl]-1-[2-[(methylsulfonyl)amino]ethyl]piperidiniumiodid;
4-[[[(2-Methoxy-1H-indol-3-yl)carbonyl]oxy]methyl]-1-methyl-1,2-[2-[(methylsulfonyl)amino]ethyl]piperidiniumiodid;

und den pharmazeutisch annehmbaren Salzen und Solvaten davon.

10. [1-[2-[(Methylsulfonyl)amino]ethyl]-4-piperidinyl]methyl-5-fluor-2-methoxy-1H-indol-3-carboxylat und die pharmazeutisch annehmbaren Salze und Solvate davon.

11. Verbindung nach Anspruch 10 in Form ihres Hydrochloridsalzes.

12. Verbindung nach Anspruch 10 in Form ihres Methansulfonatsalzes.

13. Verbindung nach Anspruch 10 in Form ihres Maleatsalzes.

14. Verbindung nach einem der Ansprüche 1 bis 13 für die Verwendung in der Therapie.

15. Verwendung einer Verbindung nach einem der Ansprüche 1 bis 13 zur Herstellung eines Arzneimittels für die Verwendung bei der Behandlung von Zuständen, die durch 5-Hydroxytryptamin vermittelt werden.

16. Pharmazeutische Zubereitung, enthaltend eine Verbindung nach einem der Ansprüche 1 bis 13 zusammen mit einem pharmazeutisch annehmbaren Träger.

17. Verfahren zur Herstellung einer Verbindung der Formel (I) nach Anspruch 1, dadurch **gekennzeichnet,** daß das Verfahren umfaßt:

(A) Umsetzung einer Verbindung der Formel (II):

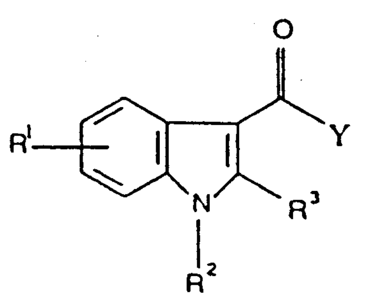

(II)

oder eines geschützten Derivats davon, worin Y ein Austrittsatom oder eine Austrittsgruppe bedeutet, mit einer Verbindung der Formel (III):

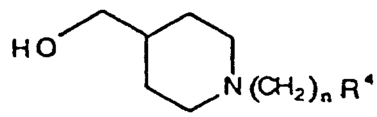 (III)

oder einem Alkalimetallalkoxid davon, worin $R^1$, $R^2$, $R^3$, $R^4$ und n die in Anspruch 1 gegebenen Definitionen besitzen;

(B) Alkylierung einer Verbindung der Formel (IV):

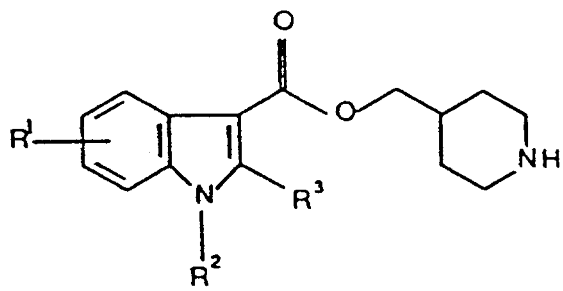 (IV)

oder eines geschützten Derivats davon mit einer Verbindung der Formel (V):

$$L(CH_2)_nR^4 \quad (V)$$

worin L ein Austrittsatom oder eine Austrittsgruppe bedeutet und $R^1$, $R^2$, $R^3$, $R^4$ und n die in Anspruch 1 gegebenen Definitionen besitzen, oder einem geschützten Derivat davon in Anwesenheit einer Base;

(C) Umwandlung einer Verbindung der Formel (I) in eine andere Verbindung der Formel (I); oder

(D) Entfernung von irgendwelchen Schutzgruppen aus der geschützten Form einer Verbindung der Formel (I).

**Patentansprüche für folgenden Vertragsstaat : ES**

1. Verfahren zur Herstellung von Verbindungen der Formel (I):

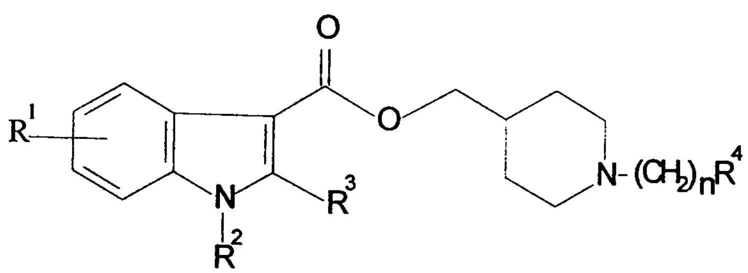 (I)

worin

$R^1$ ein Wasserstoff- oder ein Halogenatom oder eine $C_{1-6}$-Alkyl-, $C_{1-6}$-Alkoxy- oder Hydroxygruppe bedeutet;
$R^2$ ein Wasserstoffatom oder eine $C_{1-6}$-Alkyl-, $CH_2C_{2-5}$-Alkenyl- oder $CH_2C_{2-5}$-Alkinylgruppe bedeutet;
$R^3$ ein Wasserstoffatom oder eine $C_{1-6}$-Alkyl- oder $C_{1-6}$-Alkoxygruppe bedeutet;
n 2 oder 3 bedeutet;
$R^4$ eine Gruppe, ausgewählt aus Cyano, Hydroxyl, $C_{1-6}$-Alkoxy, Phenoxy, $C(O)C_{1-6}$-Alkyl, $C(O)C_6H_5$, $CONR^5R^6$, $NR^5COR^6$, $SO_2NR^5R^6$ oder $NR^5SO_2R^6$ bedeutet (wobei jeder der Substituenten $R^5$ und $R^6$ unabhängig ein Wasserstoffatom, eine $C_{1-6}$-Alkyl- oder Phenylgruppe bedeutet),

und der guaternären Ammoniumderivate, Piperidin-N-oxide und pharmazeutisch annehmbaren Salze und Solvate davon, wobei das Verfahren umfaßt:

(A) die Umsetzung einer Verbindung der Formel (II):

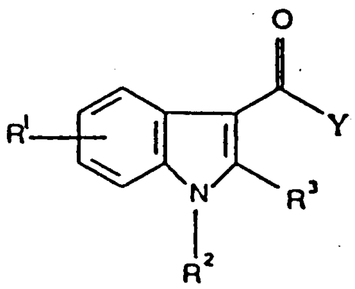

(II)

oder eines geschützten Derivats davon, worin Y ein Austrittsatom oder eine Austrittsgruppe bedeutet, mit einer Verbindung der Formel (III):

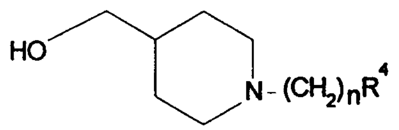

(III)

oder einem Alkalimetallalkoxid davon, worin $R^1$, $R^2$, $R^3$, $R^4$ und n die oben gegebenen Definitionen besitzen;
(B) Alkylierung einer Verbindung der Formel (IV):

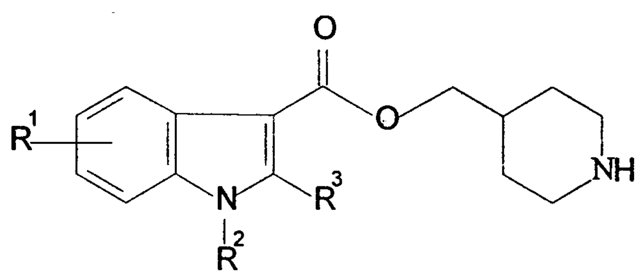

(IV)

oder eines geschützten Derivats davon mit einer Verbindung der Formel (V):

$$L(CH_2)_nR^4 \qquad (V)$$

worin L ein Austrittsatom oder eine Austrittsgruppe bedeutet und $R^1$, $R^2$, $R^3$, $R^4$ und n die oben gegebenen Definitionen besitzen, oder einem geschützten Derivat davon in Anwesenheit einer Base;
(C) Umwandlung einer Verbindung der Formel (I) in eine andere Verbindung der Formel (I); oder
(D) Entfernung von irgendwelchen Schutzgruppen aus der geschützten Form einer Verbindung der Formel (I).

2. Verfahren nach Anspruch 1, wobei die Verbindung der Formel (I) eine Verbindung der Formel (Ia):

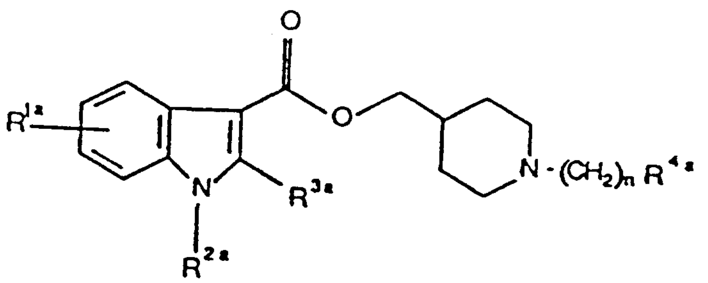

(Ia)

ist, worin

$R^{1a}$ ein Wasserstoff- oder ein Halogenatom oder eine $C_{1-6}$-Alkyl- oder $C_{1-6}$-Alkoxygruppe bedeutet;
$R^{2a}$ ein Wasserstoffatom oder eine $C_{1-6}$-Alkylgruppe bedeutet;
$R^{3a}$ ein Wasserstoffatom oder eine $C_{1-6}$-Alkyl- oder $C_{1-6}$-Alkoxygruppe bedeutet;
n 2 oder 3 bedeutet;
$R^{4a}$ eine Gruppe, ausgewählt aus CN, $C_{1-6}$-Alkoxy, $C(O)C_{1-6}$-Alkyl, $CONR^{5a}R^{6a}$, $NR^{5a}COR^{6a}$, $SO_2NR^{5a}R^{6a}$ oder $NR^{5a}SO_2R^{6a}$ bedeutet (wobei jeder der Substituenten $R^{5a}$ und $R^{6a}$ unabhängig ein Wasserstoffatom oder eine $C_{1-6}$-Alkylgruppe bedeutet),

und die quaternären Ammoniumderivate, Piperidin-N-oxide und pharmazeutisch annehmbaren Salze und Solvate davon.

3. Verfahren nach Anspruch 1 oder 2, worin $R^1$ oder $R^{1a}$ in 5-Stellung des Indolrings steht.

4. Verfahren nach einem der Ansprüche 1 bis 3, worin $R^1$ oder $R^{1a}$ ein Wasserstoff- oder Fluoratom oder eine Methylgruppe bedeutet.

5. Verfahren nach einem der Ansprüche 1 bis 4, worin $R^2$ oder $R^{2a}$ ein Wasserstoffatom oder eine Methylgruppe bedeutet.

6. Verfahren nach einem der Ansprüche 1 bis 5, worin $R^3$ oder $R^{3a}$ ein Wasserstoffatom oder eine Methoxygruppe bedeutet.

7. Verfahren nach einem der Ansprüche 1 bis 6, worin n 2 bedeutet.

8. Verfahren nach einem der Ansprüche 1 bis 7, worin $R^4$ oder $R^{4a}$ eine Gruppe $NR^5SO_2R^6$ oder $NR^{5a}SO_2R^{6a}$ bedeutet.

9. Verfahren nach Anspruch 1 zur Herstellung einer Verbindung, ausgewählt aus:

[1-[2-[(Methylsulfonyl)amino]ethyl]-4-piperidinyl]methyl-1-methyl-1H-indol-3-carboxylat;
[1-(2-Hydroxyethyl)-4-piperidinyl]methyl-1-methyl-1H-indol-3-carboxylat;
[1-[2-[(Methylamino)sulfonyl]ethyl]-4-piperidinyl]methyl-1-methyl-1H-indol-3-carboxylat;
[1-(2-Methoxyethyl)-4-piperidinyl]methyl-1-methyl-1H-indol-3-carboxylat;
[1-(3-Amino-3-oxopropyl)-4-piperidinyl]methyl-1-methyl-1H-indol-3-carboxylat;
[1-(2-Cyanoethyl)-4-piperidinyl]methyl-1-methyl-1H-indol-3-carboxylat;
[1-[2-[(Methylsulfonyl)amino]ethyl]-4-piperidinyl]methyl-1H-indol-3-carboxylat;
[1-[2-[(Methylsulfonyl)amino]ethyl]-4-piperidinyl]methyl-2-methoxy-1H-indol-3-carboxylat;
[1-[2-(Acetylamino)ethyl]-4-piperidinyl]methyl-1-methyl-1H-indol-3-carboxylat;
[1-[3-[(Methylsulfonyl)amino]propyl]-4-piperidinyl]methyl-1-methyl-1H-indol-3-carboxylat;
[1-[2-[(Methylsulfonyl)amino]ethyl]-4-piperidinyl]methyl-5-fluor-2-methoxy-1H-indol-3-carboxylat;
[1-[2-[(Methylsulfonyl)amino]ethyl]-4-piperidinyl]methyl-2-methoxy-1-methyl-1H-indol-3-carboxylat;
[1-[2-[(Phenylsulfonyl)amino]ethyl]-4-piperidinyl]methyl-1-methyl-1H-indol-3-carboxylat;
[1-[2-[(Methylsulfonyl)amino]ethyl]-4-piperidinyl]methyl-5-fluor-1-methyl-1H-indol-3-carboxylat;
[1-[2-[(Methylsulfonyl)amino]ethyl]-4-piperidinyl]methyl-5-methyl-1H-indol-3-carboxylat;

[1-[2-[(Methylsulfonyl)amino]ethyl]-4-piperidinyl]methyl-2-methoxy-5-methyl-1H-indol-3-carboxylat;
[1-[2-[Methyl(methylsulfonyl)amino]ethyl]-4-piperidinyl]methyl-1-methyl-1H-indol-3-carboxylat;
[1-[2-[(Methylsulfonyl)amino]ethyl]-4-piperidinyl]methyl-5-fluor-2-methoxy-1H-indol-3-carboxylat-N-oxid;
1-Methyl-4-[[[(1-methyl-1H-indol-3-yl)carbonyl]oxy]methyl]-1-[2-[(methylsulfonyl)amino]ethyl]piperidiniumiodid;
4-[[[(2-Methoxy-1H-indol-3-yl)carbonyl]oxy]methyl]-1-methyl-1,2-[2-[(methylsulfonyl)amino]ethyl]piperidiniumiodid;

und den pharmazeutisch annehmbaren Salzen und Solvaten davon.

**10.** Verfahren nach Anspruch 1 zur Herstellung von [1-[2-[(Methylsulfonyl)amino]ethyl]-4-piperidinyl]methyl-5-fluor-2-methoxy-1H-indol-3-carboxylat und der pharmazeutisch annehmbaren Salze und Solvate davon.

**11.** Verfahren nach Anspruch 1 zur Herstellung einer Verbindung nach Anspruch 10 in Form ihres Hydrochloridsalzes.

**12.** Verfahren nach Anspruch 1 zur Herstellung einer Verbindung nach Anspruch 10 in Form ihres Methansulfonatsalzes.

**13.** Verfahren nach Anspruch 1 zur Herstellung ener Verbindung nach Anspruch 10 in Form ihres Maleatsalzes.

**14.** Verwendung einer Verbindung der Formel (I) zur Herstellung eines Arzneimittels für die Behandlung von Zuständen, die durch 5-Hydroxytryptamin vermittelt werden.

**15.** Verfahren zur Herstellung einer pharmazeutischen Zubereitung, welches das Vermischen einer Verbindung, hergestellt nach einem der Ansprüche 1 bis 13, zusammen mit einem pharmazeutisch annehmbaren Träger umfaßt.

## Revendications

**Revendications pour les Etats contractants suivants : AT, BE, CH, DE, DK, FR, GB, GR, IT, LI, LU, MC, NL, PT, SE**

**1.** Composés de la formule (I)

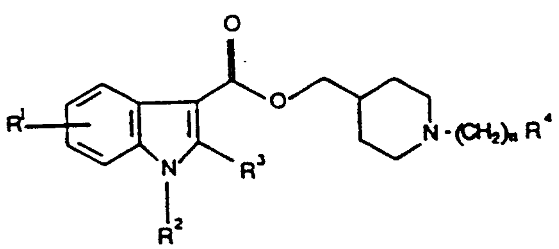

(I)

dans laquelle

$R^1$ représente un atome d'hydrogène ou un atome d'halogène, ou un radical alkyle en $C_1$ à $C_6$, alcoxy en $C_1$ à $C_6$, ou hydroxyle,
$R^2$ représente un atome d'hydrogène, ou un radical alkyle en $C_1$ à $C_6$, -$CH_2$alcényle($C_2$-$C_5$), ou -$CH_2$alcynyle($C_2$-$C_5$),
$R^3$ représente un atome d'hydrogène, ou un radical alkyle en $C_1$ à $C_6$, ou alcoxy en $C_1$ à $C_6$,
n est égal à 2 ou à 3,
$R^4$ représente un groupe choisi parmi les radicaux cyano, hydroxyle, alcoxy en $C_1$ à $C_6$, phénoxy, C(O)alkyle($C_1$-$C_6$), C(O)$C_6H_5$, -$CONR^5R^6$, -$NR^5COR^6$, -$SO_2NR^5R^6$, ou -$NR^5SO_2R^6$ (où chacun des symboles $R^5$ et $R^6$ représente indépendamment un atome d'hydrogène, un radical alkyle en $C_1$ à $C_6$, ou phényle),

et les dérivés d'ammonium quaternaire, les pipéridine N-oxydes et les solvates et sels pharmaceutiquement acceptables de ces composés.

**2.** Composés suivant la revendication 1, caractérisés en ce que les composés de la formule (I) sont des composés de la formule (Ia)

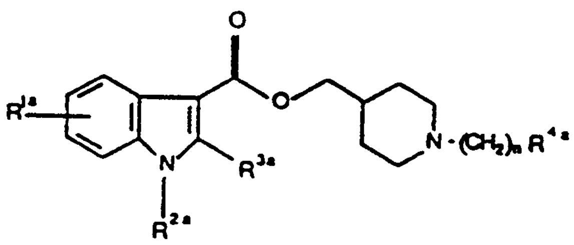

(Ia)

dans laquelle

$R^{1a}$ représente un atome d'hydrogène ou un atome d'halogène, ou un radical alkyle en $C_1$ à $C_6$, ou alcoxy en $C_1$ à $C_6$,
$R^{2a}$ représente un atome d'hydrogène, ou un radical alkyle en $C_1$ à $C_6$,
$R^{3a}$ représente un atome d'hydrogène ou un radical alkyle en $C_1$ à $C_6$, ou alcoxy en $C_1$ à $C_6$,
n est égal à 2 ou à 3,
$R^{4a}$ représente un groupe choisi parmi les radicaux CN, -alcoxy en $C_1$ à $C_6$, -C(O)alkyle($C_1$-$C_6$), -CON$R^{5a}R^{6a}$, -N$R^{5a}$COR$^{6a}$, -$SO_2$N$R^{5a}R^{6a}$ ou -N$R^{5a}SO_2R^{6a}$ (où chacun des symboles $R^{5a}$ et $R^{6a}$ représente indépendamment un atome d'hydrogène, ou un radical alkyle en $C_1$ à $C_6$); et les dérivés d'ammonium quaternaire, les pipéridine N-oxydes et les solvates et sels pharmaceutiquement acceptables de ces composés.

3. Composés suivant la revendication 1 ou la revendication 2, caractérisés en ce que $R^1$ ou $R^{1a}$ se trouve en position 5 du noyau indole.

4. Composés suivant l'une quelconque des revendications 1 à 3, caractérisés en ce que $R^1$ ou $R^{1a}$ est un atome d'hydrogène ou un atome de fluor, ou le radical méthyle.

5. Composés suivant l'une quelconque des revendications 1 à 4, caractérisés en ce que $R^2$ ou $R^{2a}$ est un atome d'hydrogène ou le radical méthyle.

6. Composés suivant l'une quelconque des revendications 1 à 5, caractérisés en ce que $R^3$ ou $R^{3a}$ est un atome d'hydrogène ou le radical méthoxy.

7. Composés suivant l'une quelconque des revendications 1 à 6, caractérisés en ce que n est égal à 2.

8. Composés suivant l'une quelconque des revendications 1 à 7, caractérisés en ce que $R^4$ ou $R^{4a}$ est un groupe $NR^5SO_2R^6$ ou $NR^{5a}SO_2R^{6a}$.

9. Composés suivant la revendication 1, choisis parmi ceux qui suivent :

[1[2[(Méthylsulfonyl)amino)éthyl]-4-pipéridinyl]méthyl-1-méthyl-1H-indole-3-carboxylate;
[1-(2-Hydroxyéthyl)-4-pipéridinyl]méthyl-1-méthyl-1H-indole-3-carboxylate;
[1-[2-(Méthylamino)sulfonyl]éthyl]-4-pipéridinyl]méthyl-1-méthyl-1H-indole-3-carboxylate;
[1-(2-Méthoxyéthyl)-4-pipéridinyl]méthyl-1-méthyl-1H-indole-3-carboxylate;
[1-(3-Amino-3-oxopropyl)-4-pipéridinyl]méthyl-1-méthyl-1H-indole-3-carboxylate;
[1-(2-Cyanoéthyl)-4-pipéridinyl]méthyl-1-méthyl-1H-indole-3-carboxylate;
[1-[2-[(Méthylsulfonyl)amino]éthyl]-4-pipéridinyl]méthyl-1H-indole-3-carboxylate;
[1-[2-[(Méthylsulfonyl)amino]éthyl]-4-pipéridinyl]méthyl-2-méthoxy-1H-indole-3-carboxylate;
[1-[2-(Acétylamino)éthyl]-4-pipéridinyl]méthyl-1-méthyl-1H-indole-3-carboxylate;
[1-[3-[(Méthylsulfonyl)amino]propyl]-4-pipéridinyl]méthyl-1-méthyl-1H-indole-3-carboxylate;
[1-[2-[(Méthylsulfonyl)amino]éthyl]-4-pipéridinyl]méthyl-5-fluoro-2-méthoxy-1H-indole-3-carboxylate;
[1-[2-[(Méthylsulfonyl)amino]éthyl]-4-pipéridinyl]méthyl-méthoxy-1-méthyl-1H-indole-3-carboxylate;
[1-[2-[(Phénylsulfonyl)amino]éthyl]-4-pipéridinyl]méthyl-1-méthyl-1H-indole-3-carboxylate;
[1-[2-[(Méthylsulfonyl)amino]éthyl]-4-pipéridinyl]méthyl-5-fluoro-1H-indole-3-carboxylate;
[1-[2-[(Méthylsulfonyl)amino]éthyl]-4-pipéridinyl]méthyl-5-méthyl-1H-indole-3-carboxylate;
[1-[2-[(Méthylsulfonyl)amino]éthyl]-4-pipéridinyl]méthyl-2-méthoxy-5-méthyl-1H-indole-3-carboxylate;
[1-[2-[Méthyl(méthylsulfonyl)amino]éthyl]-4-pipéridinyl]méthyl-1-méthyl-1H-indole-3-carboxylate;

N-Oxyde de [1-[2-[(méthylsulfonyl)amino]éthyl]-4-pipéridinyl]méthyl-5-fluoro-2-méthoxy-1H-indole-3-carboxylate;
Iodure de 1-méthyl-4-[[[(1-méthyl-1H-indol-3-yle)carbonyl]oxy]méthyl]-1-[2-[(méthylsulfonyl)amino]éthyl]pipéridinium;
Iodure de 4-[[[(2-méthoxy-1H-indol-3-yle)carbonyl]oxy]méthyl]-1-méthyl-1-2-[2-[(méthylsulfonyl)amino]éthyl]-pipéridinium;

et leurs solvates et sels pharmaceutiquement acceptables.

**10.** [1-[2-[(Méthylsulfonyl)amino]éthyl]-4-pipéridinyl]méthyl-5-fluoro-2-méthoxy-1H-indole-3-carboxylate et ses solvates et sels pharmaceutiquement acceptables.

**11.** Composé suivant la revendication 10 sous la forme de son chlorhydrate sel.

**12.** Composés suivant la revendication 10 sous la forme de son méthanesulfonate sel.

**13.** Composés suivant la revendication 10 sous la forme de son maléate sel.

**14.** Composés suivant l'une quelconque des revendications 1 à 13, destinés à l'utilisation dans le domaine thérapeutique.

**15.** Utilisation d'un composé suivant l'une quelconque des revendications 1 à 13 en vue de la préparation d'un médicament à utiliser pour le traitement d'états où intervient la 5-hydroxytryptamine.

**16.** Composition pharmaceutique, caractérisée en ce qu'elle comprend un composé suivant l'une quelconque des revendications 1 à 13, en même temps qu'un véhicule ou excipient pharmaceutiquement acceptable.

**17.** Procédé de préparation d'un composé de la formule (I) telle que définie dans la revendication 1, caractérisé en ce que :

(A) on fait réagir un composé de la formule (II) :

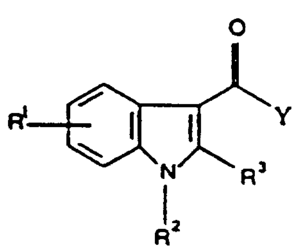

(II)

ou d'un dérivé protégé de ce composé, où Y représente un groupe ou un atome labile ou sortant, avec un composé de la formule (III) :

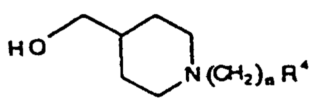

(III)

ou un alcoolate de métal alcalin de ce composés, où $R^1$, $R^2$, $R^3$, $R^4$ et n ont les significations qui leur ont été attribuées dans la revendication 1,

(B) on alkyle un composé de la formule (IV) :

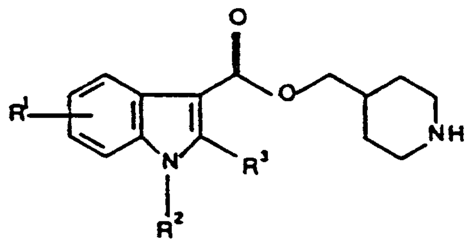

(IV)

ou un dérivé protégé de ce composé, avec un composé de la formule (V)

$$L(CH_2)_nR^4 \qquad (V)$$

dans laquelle L représente un groupe ou atome labile ou sortant et $R^1$, $R^2$, $R^3$, $R^4$ et n possèdent les significations qui leur ont été attribuées dans la revendication 1, ou un dérivé protégé de celui-ci, en présence d'une base,
(C) on convertit un composé de la formule (I) en un autre composé de la formule (I),
ou
(D) on enlève n'importe quels radicaux protecteurs d'une forme protégée d'un composé de la formule (I).

**Revendications pour l'Etat contractant suivant : ES**

1. Procédé de préparation de composés de la formule (I) :

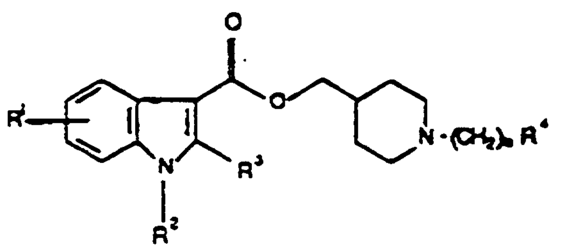

(I)

dans laquelle

$R^1$ représente un atome d'hydrogène ou un atome d'halogène, ou un radical alkyle en $C_1$ à $C_6$, alcoxy en $C_1$ à $C_6$, ou hydroxyle,
$R^2$ représente un atome d'hydrogène, ou un radical alkyle en $C_1$ à $C_6$, -$CH_2$alcényle($C_2$-$C_5$), ou -$CH_2$alcynyle($C_2$-$C_5$),
$R^3$ représente un atome d'hydrogène, ou un radical alkyle en $C_1$ à $C_6$, ou alcoxy en $C_1$ à $C_6$,
n est égal à 2 ou à 3,
$R^4$ représente un groupe choisi parmi les radicaux cyano, hydroxyle, ,alcoxy en $C_1$ à $C_6$, phénoxy, C(O)alkyle($C_1$-$C_6$), C(O)$C_6H_5$, -$CONR^5R^6$, -$NR^5COR^6$, -$SO_2NR^5R^6$, ou -$NR^5SO_2R^6$ (où chacun des symboles $R^5$ et $R^6$ représente indépendamment un atome d'hydrogène, un radical alkyle en $C_1$ à $C_6$, ou phényle),

et des dérivés d'ammonium quaternaire, des pipéridine N-oxydes et des solvates et sels pharmaceutiquement acceptables de ces composés, lequel procédé comprend :

(A) on fait réagir un composé de la formule (II) :

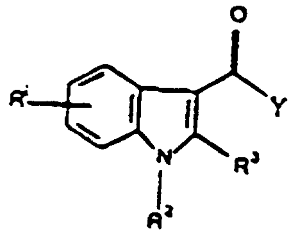

(II)

ou d'un dérivé protégé de ce composé, où Y représente un groupe ou un atome labile ou sortant, avec un composé de la formule (III) :

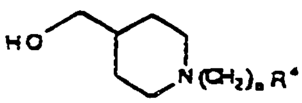

(III)

ou un alcoolate de métal alcalin de ce composée, où $R^1$, $R^2$, $R^3$, $R^4$ et n ont les significations qui leur ont été attribuées au-dessus,

(B) on alkyle un composé de la formule (IV) :

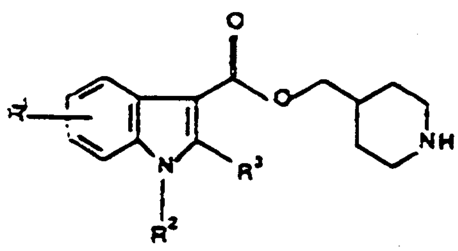

(IV)

ou un dérivé protégé de ce composé, avec un composé de la formule (V) :

$$L(CH_2)_nR^4 \qquad (V)$$

dans laquelle L représente un groupe ou atome labile ou sortant et $R^1$, $R^2$, $R^3$, $R^4$ et n possèdent les significations qui leur ont été attribuées au-dessus ou un dérivé protégé de celui-ci, en présence d'une base,

(C) on convertit un composé de la formule (I) en un autre composé de la formule (I),

ou

(D) on enlève n'importe quels radicaux protecteurs d'une forme protégée d'un composé de la formule (I).

**2.** Procédé suivant la revendication 1, caractérisé en ce que le composé de la formule (I) est un composé de la formule (Ia) :

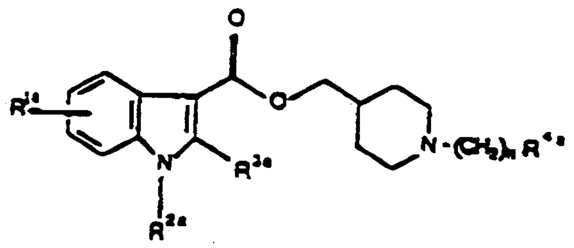

(Ia)

dans laquelle

$R^{1a}$ représente un atome d'hydrogène ou un atome d'halogène, ou un radical alkyle en $C_1$ à $C_6$, ou alcoxy en $C_1$ à $C_6$,
$R^{2a}$ représente un atome d'hydrogène, ou un radical alkyle en $C_1$ à $C_6$,
$R^{3a}$ représente un atome d'hydrogène ou un radical alkyle en $C_1$ à $C_6$, ou alcoxy en $C_1$ à $C_6$,
n est égal à 2 ou à 3,
$R^{4a}$ représente un groupe choisi parmi les radicaux CN, -alcoxy en $C_1$ à $C_6$, -C(O)alkyle($C_1$-$C_6$), -$CONR^{5a}R^{6a}$, -$NR^{5a}COR^{6a}$, -$SO_2NR^{5a}R^{6a}$ ou -$NR^{5a}SO_2R^{6a}$ (où chacun des symboles $R^{5a}$ et $R^{6a}$ représente indépendamment un atome d'hydrogène, ou un radical alkyle en $C_1$ à $C_6$); et les dérivés d'ammonium quaternaire, les pipéridine N-oxydes et les solvates et sels pharmaceutiquement acceptables de ce composé.

3. Procédé suivant la revendication 1 ou la revendication 2, caractérisé en ce que $R^1$ ou $R^{1a}$ se trouve en position 5 du noyau indole.

4. Procédé suivant l'une quelconque des revendications 1 à 3, caractérisé en ce que $R^1$ ou $R^{1a}$ est un atome d'hydrogène ou un atome de fluor, ou le radical méthyle.

5. Procédé suivant l'une quelconque des revendications 1 à 4, caractérisé en ce que $R^2$ ou $R^{2a}$ est un atome d'hydrogène ou le radical méthyle.

6. Procédé suivant l'une quelconque des revendications 1 à 5, caractérisé en ce que $R^3$ ou $R^{3a}$ est un atome d'hydrogène ou le radical méthoxy.

7. Procédé suivant l'une quelconque des revendications 1 à 6, caractérisé en ce que n est égal à 2.

8. Procédé suivant l'une quelconque des revendications 1 à 7, caractérisé en ce que $R^4$ ou $R^{4a}$ est un groupe $NR^5SO_2R^6$ ou $NR^{5a}SO_2R^{6a}$.

9. Procédé de préparation d'un composé choisi parmi ceux qui suivent :

[1-[2[(Méthylsulfonyl)amino)éthyl]-4-pipéridinyl]méthyl-1-méthyl-1H-indole-3-carboxylate;
[1-(2-Hydroxyéthyl)-4-pipéridinyl]méthyl-1-méthyl-1H-indole-3-carboxylate;
[1-[2-(Méthylamino)sulfonyl]éthyl]-4-pipéridinyl]méthyl-1-méthyl-1H-indole-3-carboxylate;
[1-(2-Méthoxyéthyl)-4-pipéridinyl]méthyl-1-méthyl-1H-indole-3-carboxylate;
[1-(3-Amino-3-oxopropyl)-4-pipéridinyl]méthyl-1-méthyl-1H-indole-3-carboxylate;
[1-(2-Cyanoéthyl)-4-pipéridinyl]méthyl-1-méthyl-1H-indole-3-carboxylate;
[1-[2-[(Méthylsulfonyl)amino]éthyl]-4-pipéridinyl]méthyl-1H-indole-3-carboxylate;
[1-[2-[(Méthylsulfonyl)amino]éthyl]-4-pipéridinyl]méthyl-2-méthoxy-1H-indole-3-carboxylate;
[1-[2-(Acétylamino)éthyl]-4-pipéridinyl]méthyl-1-méthyl-1H-indole-3-carboxylate;
[1-[3-[(Méthylsulfonyl)amino]propyl]-4-pipéridinyl]méthyl-1-méthyl-1H-indole-3-carboxylate;
[1-[2-[(Méthylsulfonyl)amino]éthyl]-4-pipéridinyl]méthyl-5-fluoro-2-méthoxy-1H-indole-3-carboxylate;
[1-[2-[(Méthylsulfonyl)amino]éthyl]-4-pipéridinyl]méthyl-2-méthoxy-1-méthyl-1H-indole-3-carboxylate;
[1-[2-[(Phénylsulfonyl)amino]éthyl]-4-pipéridinyl]méthyl-1-méthyl-1H-indole-3-carboxylate;
[1-[2-[(Méthylsulfonyl)amino]éthyl]-4-pipéridinyl]méthyl-5-fluoro-1-méthyl-1H-indole-3-carboxylate;
[1-[2-[(Méthylsulfonyl)amino]éthyl]-4-pipéridinyl]méthyl-5-méthyl-1H-indole-3-carboxylate;
[1-[2-[(Méthylsulfonyl)amino]éthyl]-4-pipéridinyl]méthyl-2-méthoxy-5-méthyl-1H-indole-3-carboxylate;
[1-[2-[Méthyl(méthylsulfonyl)amino]éthyl]-4-pipéridinyl]méthyl-1-méthyl-1H-indole-3-carboxylate;

N-Oxyde de [1-[2-[(méthylsulfonyl)amino]éthyl]-4-pipéridinyl]méthyl-5-fluoro-2-méthoxy-1H-indole-3-carboxylate;
Iodure de 1-méthyl-4-[[[(1-méthyl-1H-indol-3-yle)carbonyl]oxy]méthyl]-1-[2-[(méthylsulfonyl)amino]éthyl]pipéridinium;
Iodure de 4-[[[(2-méthoxy-1H-indol-3-yle)carbonyl]oxy]méthyl]-1-méthyl-1-2-[2-[(méthylsulfonyl)amino]éthyl] pipéridinium;

et leurs solvates et sels pharmaceutiqument acceptables.

**10.** Procédé suivant la revendication 1 pour la préparation du [1-[2-[(méthylsulfonyl)amino]éthyl]-4-pipéridinyl]méthyl-5-fluoro-2-méthoxy-1H-indole-3-car boxylate et ses solvates et sels pharmaceutiquement acceptables.

**11.** Procédé suivant la revendication 1 pour la préparation du composé de la revendication 10 sous la forme de son chlorhydrate sel.

**12.** Procédé suivant la revendication 1 pour la préparation du composé de la revendication 10 sous la forme de son méthanesulfonate sel.

**13.** Procédé suivant la revendication 1 pour la préparation du composé de la revendication 10 sous la forme de son maléate sel.

**14.** Utilisation d'un composé de la formule (I) en vue de la préparation d'un médicament à utiliser pour le traitement d'états où intervient la 5-hydroxytryptamine.

**15.** Procédé de préparation d'une composition pharmaceutique, caractérisé en ce que l'on mélange un composé suivant l'une quelconque des revendications 1 à 13 et un véhicule ou excipient pharmaceutiquement acceptable.